# FASCICULE DE BREVET EUROPEEN

(11) **EP 2 793 959 B1**
(45) Date de publication et mention de la délivrance du brevet: **30.04.2025**
(21) Numéro de dépôt: 12801773.8
(22) Date de dépôt: 18.12.2012
(51) Int. Cl.: A61L 2/232, A61L 2/18

(54) **GEL DE DÉCONTAMINATION ET PROCÉDÉ DE DÉCONTAMINATION DE SURFACES PAR IMMERSION UTILISANT CE GEL**
DEKONTAMINATIONSGEL UND VERFAHREN ZUR DEKONTAMINATION VON OBERFLÄCHEN DURCH EINTAUCHEN IN DIESEM GEL
DECONTAMINATION GEL AND METHOD FOR DECONTAMINATING SURFACES BY IMMERSION USING SAID GEL

(30) Priorité: 19.12.2011 FR 1161959
(43) Date de publication de la demande: 29.10.2014
(73) Titulaire: Commissariat à l'Energie Atomique et aux Energies Alternatives, 75015 Paris (FR); Orano Démantèlement, 92320 Chatillon (FR)
(72) Inventeur: CUER, Frédéric, F-30630 Cornillon (FR); CASTELLANI, Romain, F-30200 Bagnols sur Ceze (FR)
(74) Mandataire: Brevalex
(86) Numéro de dépôt international: PCT/EP2012/076026
(87) Numéro de publication internationale: WO 2013/092633

(56) Documents cités:
- WO-A1-2010/037809
- WO-A1-2012/001046
- WO-A1-97/35323
- WO-A2-2007/142967
- WO-A2-2010/079487
- SHCHIPUNOV ET AL: "Sol-gel-derived biomaterials of silica and carrageenans", JOURNAL OF COLLOID AND INTERFACE SCIENCE, ACADEMIC PRESS,INC, US, vol. 268, no. 1, 1 December 2003 (2003-12-01), pages 68 - 76, XP027432870, ISSN: 0021-9797, [retrieved on 20031201], DOI: 10.1016/S0021-9797(03)00457-0

## Description

### DOMAINE TECHNIQUE

La présente invention a pour objet un gel de décontamination utilisable pour la décontamination de surfaces.

Le gel selon l'invention est particulièrement adapté à une application sur les surfaces contaminées de pièces par trempage, immersion et à la décontamination des surfaces contaminées de pièces de petites tailles et/ou de formes complexes.

La présente invention a trait, en outre, à un procédé de décontamination des surfaces contaminées de pièces dans lequel on applique un gel sur les surfaces contaminées des pièces par trempage, immersion.

Le procédé et le gel selon l'invention permettent la décontamination de toutes sortes de surfaces telles que les surfaces en matériaux métalliques, plastiques, minéraux comme les matériaux vitreux.

Le procédé et le gel selon l'invention s'appliquent tout particulièrement à la décontamination des surfaces de matériaux poreux tels que les matériaux cimentaires comme les mortiers et les bétons ; les briques ; les plâtres ; et la pierre naturelle.

Le procédé et le gel selon l'invention permettent également l'élimination de toutes sortes de contaminants et notamment des contaminants chimiques, biologiques, ou nucléaires, radioactifs.

Le domaine technique de l'invention peut ainsi, de manière générale, être défini comme étant celui de la décontamination de surfaces en vue d'éliminer les polluants, contaminants qui se trouvent sur cette surface et éventuellement sous cette surface, et dont la présence sur et sous ces surfaces n'est pas souhaitée.

### ÉTAT DE LA TECHNIQUE ANTÉRIEURE

Depuis une vingtaine d'années, de nombreux procédés de décontamination de matériaux solides ont été développés pour répondre aux besoins de l'industrie nucléaire notamment du fait que celle-ci se trouve dans une phase de mise à l'arrêt et de démantèlement des installations de première génération.

Par ailleurs, la succession d'actes terroristes par voie chimique ou biologique incite de nombreux pays à bâtir de véritables programmes de recherche pour se munir de moyens de décontamination efficaces des biens et des personnes.

D'une façon générale, les techniques d'assainissement les plus faciles à mettre en œuvre et les moins onéreuses consistent en la mise en contact d'un liquide contenant un réactif chimique, que l'on peut appeler solution décontaminante, avec les surfaces contaminées par exemple par une contamination biologique.

L'application de la solution décontaminante est généralement réalisée par pulvérisation ou par lavage couplé ou non à un effet mécanique tel qu'un brossage.

Ainsi, le document [1] décrit-il une composition de nettoyage pour éliminer les agents antibactériens et autres utilisés dans la décontamination suite à une attaque biologique. Cette composition comprend notamment de l'éthanol, de l'isopropanol, de l'éther n-hexylique de l'éthylèneglycol, un bromure et un chlorure.

Le document [2] décrit un procédé de décontamination à grande échelle dans lequel un peracide solide, stable, ou une source solide, stable, d'un peracide est mis en contact avec une surface contaminée.

Plus récemment, de nouvelles techniques de mouillage par nébulisation ou par projection de mousse ont permis de réduire la quantité de produit décontaminant utilisée et donc le volume d'effluents chimiques produits. On pourra à cet égard se référer aux documents [3] et [4].

D'autres procédés, utilisent des réactifs chimiques sous forme gazeuse, comme le procédé décrit dans le document [5] qui met en œuvre des agents biocides, sous forme gazeuse, tels que le peroxyde d'hydrogène ou encore l'ozone.

Quelle que soit la technique utilisée, le risque majeur associé aux procédés décrits dans ce qui précède, reste toutefois le risque de dissémination d'agents toxiques dans l'environnement.

En outre, le procédé qui met en œuvre des agents biocides sous forme gazeuse, est exclusivement efficace lors d'une mise en œuvre dans des enceintes closes.

Pour répondre à la problématique de récupération de la contamination, une troisième catégorie de procédé a plus récemment été développée.

Dans ces procédés, le transfert de la contamination se fait vers un matériau support solide capable de piéger par sorption les contaminants, voire même de les détruire dans le cas des espèces biologiques toxiques. Le déchet ainsi généré se retrouve alors également sous forme solide. L'obtention d'un déchet solide est particulièrement intéressante pour limiter les risques de dispersion des agents toxiques dans l'environnement mais aussi pour faciliter la gestion et le traitement ultime du déchet produit.

Différentes technologies mettant en œuvre un matériau support solide ont d'ores et déjà été développées.

Il s'agit tout d'abord de la technologie dite du « gant poudreur » destinée à la décontamination de liquides toxiques persistants se trouvant sur la peau ou sur des équipements.

Dans ce gant, l'agent décontaminant est une poudre absorbante, généralement de la Terre de foulon.

La mise en œuvre de ce gant est réalisée en trois étapes successives :
- application de la poudre sur la zone contaminée du substrat (peau ou matériau) par tapotage ;
- absorption du liquide toxique ;
- essuyage, nettoyage du substrat à l'aide de la face éponge du gant [6].

Dans le cas d'une contamination comprenant des agents pathogènes, le gant poudreur peut également contenir un agent oxydant capable d'inactiver cette contamination piégée par la Terre de foulon.

Cette technique, particulièrement adaptée au soin des personnes, reste néanmoins limitée au traitement des contaminations liquides de faible envergure.

D'autres produits de décontamination, qui se présentent sous la forme d'un gel, génèrent un déchet solide et permettent ainsi de s'affranchir de l'utilisation de solutions liquides pour traiter, assainir des pièces de grandes surfaces et de géométries complexes.

Ces gels sont généralement mis en œuvre en les pulvérisant sur la surface à décontaminer.

Après un temps de contact du gel avec la surface à décontaminer, équivalent à la durée d'évaporation du solvant, le déchet sec obtenu est éliminé par brossage et/ou aspiration. L'intérêt majeur de ces procédés est leur aptitude au traitement des grandes surfaces et de géométries accidentées.

Ainsi, le document [7] décrit une composition de gel contenant des agents oxydants pour la décontamination chimique ou biologique de zones contaminées. Cette composition est préparée en ajoutant des agents épaississants ou gélifiants sous la forme de colloïdes à une solution d'agent oxydant pour former un gel colloïdal visqueux.

Cette solution peut être une solution aqueuse ou organique.

Les agents épaississants ou gélifiants peuvent être choisis parmi la silice, l'alumine, les aluminosilicates, les mélanges de silice et d'alumine, et les argiles telles que la smectite.

Les agents oxydants sont notamment l'hypochlorite de sodium, le persulfate d'ammonium, ou le peroxyde d'hydrogène.

Il est indiqué que ces gels peuvent être utilisés pour éliminer des agents biologiques tels que des micro-organismes comme les bactéries, champignons, virus, et spores, ou des agents chimiques tels que les gaz neurotoxiques.

Les gels sont ensuite pulvérisés sur les surfaces à traiter puis récupérés par aspiration après séchage.

Les formulations gélifiées développées par le Lawrence Livermore National Laboratory sous les noms de L-Gel 115, et L-Gel 200 sont analogues aux formulations développées dans le document [7] et sont mises en œuvre avec le procédé dit « L-Gel ». Ces gels sont formulés à partir de solutions acides oxydantes auxquelles sont ajoutés des solvants organiques et une charge de silice. Les gels sont ensuite pulvérisés sur les surfaces à traiter puis récupérés par aspiration après séchage. Parmi les points critiques de ce procédé, on peut citer les propriétés rhéologiques du produit gélifié.

En effet, afin d'éviter les coulures, en particulier lorsque le gel est appliqué sur les murs ou les plafonds, celui ci est appliqué sous forme de films très minces d'une épaisseur ne dépassant pas, dans le document [7], 125 µm. Il en résulte un déchet sec pulvérulent pouvant entraîner, si l'efficacité du traitement n'est pas totale, une dissémination des espèces bio-toxiques et chimiques, telles que les composés oxydants, dans l'atmosphère.

Les performances du procédé semblent toutefois très modérées vis-à-vis des contaminations biologiques et nucléaires.

Ainsi les performances du procédé, déterminées vis-à-vis d'une contamination par l'Anthrax sous forme d'aérosol déposé par pulvérisation sur des matériaux de références (10⁷ et 10⁸ spores par échantillon de 0,16 m² soit 6000 à 63000 spores/cm²), montrent qu'il n'autorise pas une réduction de la contamination supérieure à 5 décades [8].

Il n'existe cependant pas de bilan précis sur le rendement de ce procédé vis-à-vis d'une contamination nucléaire.

Par ailleurs, dans le cadre de la décontamination nucléaire, des formulations gélifiées qui permettent de s'affranchir des problèmes liés au caractère pulvérulent du déchet sec et d'accroître l'efficacité du procédé gel ont fait l'objet des documents [9] et [10]. Les formulations de gel qui y sont décrites permettent de libérer, au moyen d'une faible érosion du matériau support, la contamination sous forme particulaire. Le contaminant se retrouve alors piégé dans le résidu de gel sec [9], [10].

Ces gels ont été mis en œuvre avec succès sur différents chantiers de décontamination nucléaire. Les facteurs de réduction du débit de dose obtenus sont très largement supérieurs à 20 y compris sur les matériaux résistants tels que l'acier inoxydable. Ce type de traitement présente également l'avantage de respecter les critères d'innocuité des matériaux en vue de leur réutilisation.

Cependant les gels décrits dans les documents de l'art antérieur, tels que les gels aspirables décrits dans les documents [9] et [10] malgré tous leurs avantages, ne sont pas compatibles avec la décontamination de pièces de petites tailles et/ou de formes, de géométries complexes.

En effet, la décontamination de ces pièces de petites tailles et/ou de formes, de géométries complexes est généralement réalisée par un procédé de décontamination dit procédé de trempage ou procédé d'immersion qui consiste en une simple immersion des pièces contaminées dans un bain décontaminant.

Pour des pièces de faible volume, le procédé d'immersion permet à la fois de limiter le temps d'intervention des opérateurs, mais aussi d'assurer la présence de l'agent décontaminant en tout point de la pièce, y compris dans les zones les plus difficiles d'accès.

Or, les inventeurs ont mis en évidence que les gels décrits dans les documents de l'art antérieur ne sont pas adaptés à une mise en œuvre dans un tel procédé de trempage.

Les bains utilisés dans ces procédés de trempage sont actuellement des solutions liquides qui présentent des risques élevés d'épandage ou de coulures lorsque la pièce est extraite du bain.

Il existe donc, au regard de ce qui précède, un besoin pour un gel de décontamination qui puisse être mis en œuvre de manière satisfaisante dans un procédé de décontamination par trempage ou par immersion et qui puisse ainsi permettre la décontamination de pièces de petites tailles et/ou de formes, de géométries complexes.

En d'autres termes, il existe un besoin pour un gel de décontamination qui tout en présentant toutes les excellentes propriétés des gels de décontamination de l'art antérieur et en particulier des gels de décontamination aspirables décrits dans les documents [9] et [10] n'en présente pas les inconvénients notamment en ce qui concerne leur inaptitude à une mise en œuvre dans un procédé de décontamination par trempage ou par immersion.

Le but de la présente invention est de fournir un gel de décontamination qui réponde entre autres à ce besoin.

Le but de la présente invention peut aussi être défini comme l'amélioration des gels et procédés de l'art antérieur et notamment ceux des documents [9] et [10] afin de les rendre compatibles avec la décontamination des pièces de petites tailles et/ou de géométries complexes par un procédé de décontamination par trempage ou par immersion.

Un autre but de la présente invention est de fournir un gel de décontamination qui permette la décontamination efficace de surfaces poreuses notamment minérales et qui assure la décontamination, notamment biologique, en profondeur de telles surfaces sur une profondeur pouvant atteindre par exemple plusieurs millimètres.

### EXPOSÉ DE L'INVENTION

Ce but, et d'autres encore, sont atteints, conformément à l'invention, par un gel de décontamination tel que défini dans le jeu de revendications annexé, constitué par une solution colloïdale comprenant ou constituée par :
- 0,1% à 30% en masse, de préférence 0,1% à 25% en masse, de préférence encore 5% à 25% en masse, mieux 8% à 20% en masse, par rapport à la masse du gel, d'au moins un agent viscosant inorganique ;
- 0,01% à 5% en masse, de préférence 0,05% à 3% en masse, par rapport à la masse du gel, d'au moins un agent de variation de la viscosité qui, lorsqu'il est soumis à au moins un stimulus, de préférence externe au gel, provoque une variation de la viscosité du gel,
   ledit stimulus, de préférence externe au gel, étant choisi parmi les stimuli chimiques tels qu'une variation du potentiel d'oxydoréduction du gel ; thermiques tels qu'une variation de la température du gel ; physiques tels qu'une exposition à une onde électromagnétique ; et les combinaisons de deux ou plus desdits stimuli ;
   ledit agent de variation de la viscosité n'étant pas un polymère super-absorbant;
   ledit agent de variation de la viscosité étant choisi dans le groupe constitué par les polysaccharides, les composés oxydants, les particules magnétiques et les composés dont la viscosité varie lorsqu'ils sont exposés à des radiations électromagnétiques de longueurs d'ondes différentes ;
- 0,1 à 10 mol/L de gel, de préférence 0,5 à 10 mol/L de gel, de préférence encore 1 à 10 mol/L de gel, d'au moins un agent actif de décontamination différent dudit agent de variation de la viscosité ;
- 0,1% à 2% en masse par rapport à la masse du gel, d'au moins un agent tensio-actif et de préférence d'au moins un agent tensio-actif non ionique ;
- éventuellement 0,05% à 5% en masse, de préférence 0,05% à 2% en masse par rapport à la masse du gel, d'au moins un polymère super-absorbant ;
- et le reste de solvant.

Le gel selon l'invention contient un agent de variation de la viscosité qui, lorsqu'il est soumis à au moins un stimulus, de préférence externe au gel, provoque une variation de la viscosité du gel, de préférence une augmentation de la viscosité du gel ou saut de viscosité.

De préférence, cette variation de la viscosité du gel est une variation, et notamment une augmentation, que l'on peut qualifier de rapide et d'importante.

Cet agent de variation de la viscosité peut aussi être dénommé agent générant un comportement rhéologique versatile.

En d'autres termes, le gel selon l'invention comprend un ou plusieurs agents qui sont capables d'induire une variation, évolution, généralement importante de la viscosité du gel.

Cette évolution de la viscosité doit généralement être brutale et est consécutive à un stimulus externe au gel généré de façon simple.

Par évolution rapide, brutale, importante de la viscosité du gel, par exemple augmentation rapide, brutale, importante, on entend généralement que la viscosité du gel subit une variation, de préférence une augmentation de 0,05 Pa.s à 10⁵ Pa.s en une durée inférieure à 5 secondes, de préférence inférieure à 2 secondes, de préférence encore inférieure ou égale à 1 seconde, par exemple en une durée comprise entre 0,5 seconde et 1 seconde à partir de l'instant où le gel est soumis audit stimulus.

Généralement, le gel passe d'un état que l'on peut qualifier de liquide avec une viscosité inférieure à 0,1 Pa.s à un état que l'on peut qualifier véritablement de gélifié avec une viscosité supérieure à 100 Pa.s.

Ledit stimulus, de préférence externe au gel, est choisi parmi les stimuli chimiques tels qu'une variation du potentiel d'oxydoréduction du gel ; thermiques tel qu'une variation de la température du gel ; physiques tel qu'une exposition à une onde électromagnétique ; et les combinaisons de deux ou plus desdits stimuli.

Dans le cas où le stimulus est un stimulus chimique, il peut s'agir d'une variation du potentiel d'oxydoréduction du gel.

On peut par exemple formuler un gel de décontamination oxydant dont la viscosité augmente brutalement au contact d'un réducteur constitué par exemple par le matériau de la pièce à décontaminer.

Dans le cas où le stimulus est une variation du potentiel d'oxydoréduction du gel, l'agent de variation de la viscosité est choisi parmi les composés oxydants tels que les ions Ce IV, H₂O₂, KMnO₄ etc.

Dans le cas où le stimulus est un stimulus chimique, il peut s'agir aussi d'une variation de l'acidité du gel. Dans ce cas, l'agent de variation de la viscosité peut être choisi parmi les carraghénanes dont le mécanisme de gélification est sensible à l'acidité. Comme on le mentionne plus bas, le mécanisme de gélification des carraghénanes est aussi sensible à la température.

Dans le cas où le stimulus est un stimulus thermique, il peut s'agir d'une variation de la température du gel et l'agent de variation de la viscosité est alors choisi parmi les agents viscosants organiques dont la viscosité varie en fonction de la température.

Ces agents viscosants organiques, sensibles à la température, dont la viscosité varie en fonction de la température, sont choisis parmi les polysaccharides, tels que les carraghénanes.

Les carraghénanes présentent en effet, en milieu aqueux, des variations de viscosité importantes en fonction de la température, leur viscosité diminuant généralement de manière importante en fonction de la température (Exemple 6).

Les carraghénanes sont des gélifiants ou épaississants communément utilisés dans l'industrie alimentaire et issus d'algues rouges.

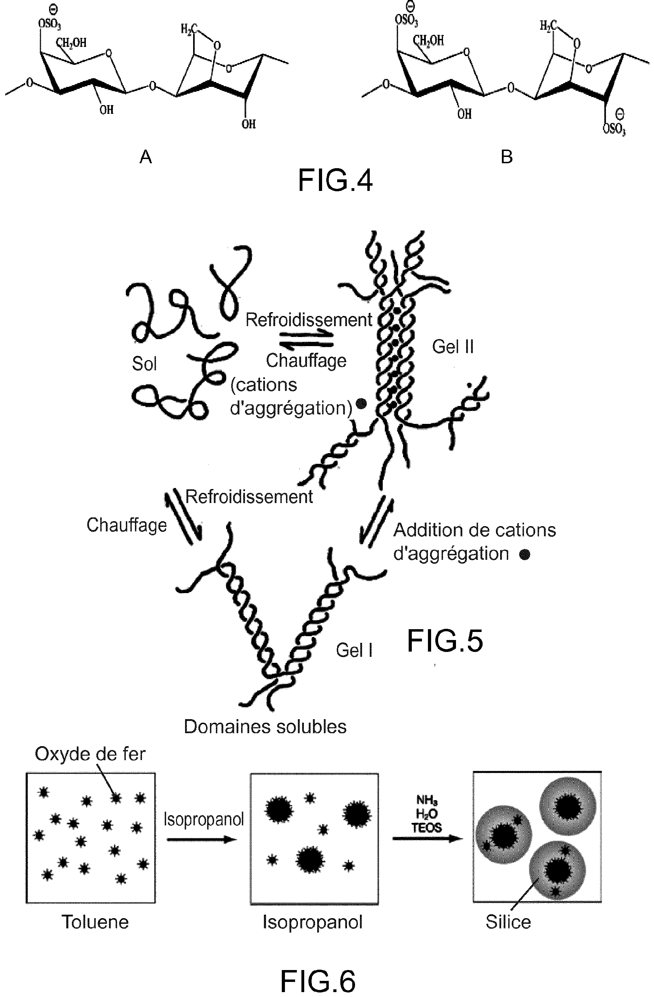

La faculté de ces produits à se gélifier dépend du nombre et de la position des groupements sulfonates sur les cycles carbonés (Figures 4 A et B) [13].

Nous nous intéressons préférentiellement dans la présente aux formes 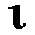 (iota) (Figure 4B) et κ (kappa) (Figure 4A) réputées pour former respectivement des gels « souples » et des gels « cassants ».

Le mécanisme de gélification de ce type de produit fait intervenir un changement de conformation des chaînes carbonées ; enchevêtrées de façon désordonnée à haute température, elles s'organisent en hélices une fois le point de transition sol-gel atteint (Figure 5) [14].

Ainsi, un gel selon l'invention contenant des carraghénanes, de préférence des carraghénanes κ et 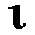 forme-t-il à une température de 30°C à 80°C, par exemple de 40°C à 65°C, un bain liquide et peu visqueux dans lequel la pièce à décontaminer est immergée. Lorsque la pièce nappée d'un film de ce gel est sortie du bain chauffé, le gel subit une brusque diminution de température par exemple jusqu'à une température de 15°C à 25°C, et voit sa viscosité rapidement augmenter en passant d'un état liquide à un état véritablement gélifié.

La teneur en agents viscosants organiques, tels que des carraghénanes, dont la viscosité varie en fonction de la température est généralement comprise entre 0% et 5%, de préférence entre 0,1% et 5% en masse de gel, de préférence encore entre 0,1% et 2% en masse de la masse totale du gel.

Dans ces proportions, la nature organique de l'agent de variation de la viscosité n'est en aucun cas préjudiciable à l'utilisation du produit décontaminant dans un contexte nucléaire où les risques de dégradation des déchets par radiolyse doivent nécessairement être pris en compte.

Dans le cas où le stimulus est un stimulus physique, il peut s'agir de l'exposition ou non du gel à un champ électromagnétique.

L'agent de variation de la viscosité est alors choisi parmi les particules magnétiques.

En effet, les transitions de viscosité requises par le procédé de la présente invention décrit plus bas peuvent être induites par la présence de particules magnéto-sensibles dans la composition du gel.

Les suspensions de particules magnétiques de taille micrométrique présentent une grande différence de comportement rhéologique lorsque le fluide complexe est soumis ou non à un champ magnétique.

En s'alignant sous l'effet du champ, les agglomérats solides contenant les particules magnétiques induisent une résistance au cisaillement non négligeable, d'où l'évolution de leur viscosité [15].

En choisissant le sens d'alignement des particules par l'intermédiaire du champ magnétique, on peut soit augmenter ou au contraire réduire la résistance au cisaillement, donc favoriser l'apparition d'une phase liquide ou bien générer la gélification. Cela revient à dire que ledit stimulus peut consister soit au début de l'exposition du gel à un champ magnétique, soit à la fin de l'exposition du gel à un champ magnétique.

Plusieurs procédés de synthèse permettent d'obtenir des composites fer/silice sous forme de particules contenant un noyau d'oxyde de fer magnétique et présentant un comportement magnéto-rhéologique [16], [17].

Le document [18] décrit un mode opératoire qui permet d'obtenir des particules de silice ferromagnétiques qui peuvent être utilisées dans les gels selon l'invention (Figure 6).

A titre d'exemple, on peut également citer le produit vendu par la société EVONIK^{®} sous la désignation commerciale « *MagSilica* », qui est un oxyde de fer nanostructuré dans une matrice de silice.

Dans le cadre de l'application visée, ce type d'adjuvant est particulièrement intéressant puisqu'il est directement compatible avec la silice qui peut être utilisée comme charge minérale viscosante dans le gel selon l'invention.

Toujours dans le cas où le stimulus est un stimulus physique, il peut s'agir de l'exposition du gel à un rayonnement, dans la gamme des rayonnements infrarouges à ultraviolet, ce rayonnement induisant une variation de la viscosité du gel.

L'agent de variation de la viscosité est alors choisi parmi les composés dont la viscosité varie lorsqu'ils sont exposés à des rayonnements de longueurs d'ondes différentes. On est donc en présence d'un stimulus que l'on peut plus précisément qualifier de physico-chimique.

Ainsi, les rayonnements ultra-violets peuvent être utilisés comme stimuli externes activant un adjuvant photosensible. Le gel de nappage selon l'invention peut alors comprendre un adjuvant dont le comportement rhéologique est fonction d'au moins deux longueurs d'ondes (l'une pour l'état liquide, l'autre pour l'état gélifié) [19].

La variation de la viscosité du gel peut également intervenir suite à la combinaison de plusieurs stimuli choisis parmi les stimuli énumérés plus haut, par exemple la combinaison de plusieurs stimuli choisis parmi un stimulus physique, un stimulus chimique par exemple une variation du potentiel d'oxydoréduction, et un stimulus physicochimique par exemple une exposition à un rayonnement UV.

Cette combinaison de sollicitations externes pour passer d'un état rhéologique à un autre est notamment décrite dans les documents [20] et [21].

Les gels selon l'invention n'ont jamais été décrits dans l'art antérieur.

En particulier, l'incorporation dans un gel de décontamination, d'un agent de variation de la viscosité qui, lorsqu'il est soumis à au moins un stimulus externe au gel, provoque une variation de la viscosité du gel, de préférence une augmentation importante et rapide de la viscosité du gel, et *a fortiori* la combinaison dans un tel gel d'un tel agent de variation de la viscosité avec un agent de décontamination tel qu'un agent de décontamination biologique, n'ont jamais été décrites dans l'art antérieur, tel que représenté notamment par les documents cités plus haut.

L'incorporation dans un gel de décontamination d'un agent de variation de la viscosité n'a en outre jamais été suggérée dans l'art antérieur tel que représenté notamment par les documents cités plus haut.

Les gels selon l'invention répondent à l'ensemble des besoins mentionnés plus haut, ils ne présentent pas les inconvénients, défauts, limitations et désavantages des gels de l'art antérieur tels que ceux décrits dans les documents mentionnés plus haut.

Les gels selon l'invention résolvent les problèmes présentés par les gels de décontamination de l'art antérieur tels que ceux décrits dans les documents mentionnés plus haut, et notamment les gels des documents [9] et [10] sans en présenter les inconvénients mais tout en conservant toutes les propriétés avantageuses connues de ces gels.

La formulation du gel selon l'invention, notamment du fait de la présence dans ce gel, d'un agent de variation de la viscosité permet, de manière surprenante, l'utilisation du gel selon l'invention dans un procédé de décontamination, dit procédé de décontamination par immersion ou trempage, qui consiste en une simple immersion des pièces contaminées dans un bain décontaminant.

De ce fait, le gel selon l'invention peut être dénommé « gel de nappage », puisque la pièce à décontaminer est nappée d'un film du gel selon l'invention, par trempage, immersion dans un bain.

Les gels décrits dans les documents de l'art antérieur qui ne contiennent pas un tel agent de variation de la viscosité ne sont absolument pas adaptés à une mise en œuvre dans un procédé de décontamination par immersion ou trempage. L'utilisation d'un gel dans un tel procédé est rendue pour la première fois possible grâce à la composition spécifique du gel selon l'invention, et c'est-là un des problèmes essentiels des gels de l'art antérieur qui se trouve donc surmonté par le gel selon l'invention.

Le gel selon l'invention remplace les solutions liquides qui sont habituellement utilisées pour les bains de trempage et n'en présente pas les inconvénients notamment en ce qui concerne les coulures observées avec ces bains de trempage liquide lorsque la pièce, qui a été immergée dans le bain de trempage liquide, et qui est donc nappée par la solution liquide est sortie de celui-ci.

Le gel qui forme le bain dans lequel la pièce à décontaminer est immergée présente une faible viscosité et se trouve dans un état que l'on peut qualifier de liquide. Du fait que le gel est peu visqueux, il peut atteindre tous les points de la surface de la pièce à décontaminer y compris les zones les plus difficiles d'accès des pièces qui présentent une géométrie complexe.

Autrement dit, le gel de décontamination se comporte comme un liquide dans le bain de trempage et peut ainsi s'infiltrer dans toutes les parties de la pièce et recouvrir toutes les surfaces des pièces contaminées et même les surfaces les plus difficiles d'accès.

Le gel selon l'invention est ainsi particulièrement adapté au traitement de pièces de petite taille et/ou de forme, géométrie complexe.

Dès que la pièce nappée de gel est sortie, extraite du bain, on soumet le film de gel qui nappe la pièce, à l'extérieur du bain, au stimulus décrit plus haut ce qui provoque à la sortie du bain une augmentation très rapide, voire quasiment instantanée de la viscosité du film de gel qui nappe la pièce. Le film de gel possède alors généralement une viscosité suffisante garantissant l'adhésion du film de gel et évitant les coulures.

Autrement dit, le gel qui était à l'état liquide dans le bain, retrouve une structure de type gel dès que la pièce est sortie du bain.

Un des inconvénients majeurs des bains de trempage liquides de l'art antérieur dans lesquels aucun changement de viscosité ne se produit lorsque la pièce est sortie du bain est ainsi surmonté.

Le gel selon l'invention peut éventuellement comprendre un polymère super-absorbant.

L'incorporation d'un polymère super-absorbant dans un gel de décontamination et *a fortiori* la combinaison dans un tel gel d'un tel polymère super-absorbant avec un agent de décontamination, tel qu'un agent de décontamination biologique, et avec un agent de variation de la viscosité n'ont jamais été décrites dans l'art antérieur, tel que représenté notamment par les documents cités plus haut.

Le gel selon l'invention est une solution colloïdale, ce qui signifie que le gel selon l'invention contient des particules solides inorganiques, minérales, d'agent viscosant dont les particules élémentaires, primaires, ont une taille généralement de 2 à 200 nm.

Du fait de la mise en œuvre d'un agent viscosant généralement exclusivement inorganique, sans agent viscosant organique, la teneur en matières organiques du gel selon l'invention est généralement inférieure à 4% en masse, de préférence inférieure à 2% en masse, ce qui constitue encore un autre avantage des gels selon l'invention.

Ces particules solides, minérales, inorganiques jouent le rôle de viscosant pour permettre à la solution, par exemple la solution aqueuse, de se gélifier et ainsi d'adhérer aux surfaces de la pièce à traiter, décontaminer, quelle que soit leur géométrie, leur forme, leur taille, et où que se trouvent les contaminants à éliminer (voir Exemple 6) .

Avantageusement, l'agent viscosant inorganique peut être choisi parmi les alumines, les silices, les aluminosilicates, les argiles telles que la smectite, et leurs mélanges.

En particulier, le viscosant inorganique peut être choisi parmi les alumines (Al₂O₃) et les silices (SiO₂).

Le viscosant inorganique peut ne comprendre qu'une seule silice ou alumine ou un mélange de celles-ci, à savoir un mélange de deux silices différentes ou plus (mélange SiO₂/SiO₂), un mélange de deux alumines, différentes ou plus (mélange Al₂O₃/Al₂O₃), ou encore un mélange d'une ou plusieurs silices avec une ou plusieurs alumines (mélange SiO₂/Al₂O₃).

Avantageusement, l'agent viscosant inorganique peut être choisi parmi les silices pyrogénées, les silices précipitées, les silices hydrophiles, les silices hydrophobes, les silices acides, les silices basiques comme la silice Tixosil^{®} 73, commercialisée par la société Rhodia, et leurs mélanges.

Parmi les silices acides, on peut notamment citer les silices pyrogénées ou fumées de silice "Cab-O-Sil"^{®} M5, H5 ou EH5, commercialisées par la société CABOT, et les silices pyrogénées commercialisées par la société DEGUSSA sous l'appellation AEROSIL^{®}.

Parmi ces silices pyrogénées, on préférera encore la silice AEROSIL^{®} 380 d'une surface spécifique de 380 m²/g qui offre les propriétés viscosantes maximales pour une charge minérale minimale.

La silice utilisée peut aussi être une silice dite précipitée obtenue par exemple par voie humide par mélange d'une solution de silicate de soude et d'un acide. Les silices précipitées préférées sont commercialisées par DEGUSSA sous le nom de SIPERNAT^{®} 22 LS et FK 310 ou encore par la société RHODIA sous le nom de TIXOSIL^{®} 331, cette dernière est une silice précipitée dont la surface spécifique moyenne est comprise entre 170 et 200 m²/g.

Avantageusement, l'agent viscosant inorganique est constitué par un mélange d'une silice précipitée et d'une silice pyrogénée.

L'alumine peut être choisie parmi les alumines calcinées, les alumines calcinées broyées, et leurs mélanges.

A titre d'exemple, on peut citer le produit vendu par DEGUSSA sous la désignation commerciale « Aeroxide Alumine C » qui est de l'alumine fine pyrogénée.

De manière avantageuse, selon l'invention, l'agent viscosant est constitué par une ou plusieurs alumine(s) représentant généralement de 5% à 30% en masse par rapport à la masse du gel.

Dans ce cas, l'alumine est de préférence à une concentration de 8% à 17% en masse par rapport à la masse totale du gel pour assurer un séchage du gel à température comprise entre 20°C et 50°C et à une humidité relative comprise entre 20% et 60% en moyenne en 30 minutes à 5 heures.

La nature de l'agent viscosant minéral, notamment lorsqu'il est constitué d'une ou plusieurs alumine(s), influence de manière inattendue le séchage du gel selon l'invention et la granulométrie du résidu obtenu.

En effet, le gel sec se présente sous la forme de particules de taille contrôlée, plus précisément de paillettes solides millimétriques, dont la taille va généralement de 1 à 10 mm, de préférence de 2 à 5 mm grâce notamment aux compositions précitées de la présente invention, en particulier lorsque l'agent viscosant est constitué par une ou plusieurs alumine(s).

Précisons que la taille des particules correspond généralement à leur plus grande dimension.

En d'autres termes, les particules solides minérales du gel selon l'invention, par exemple de type silice ou alumine, outre leur rôle de viscosant, jouent également un rôle fondamental lors du séchage du gel car elles assurent la fracturation du gel pour aboutir à un déchet sec sous forme de paillettes (Exemple 7).

Le gel selon l'invention contient un agent actif de décontamination. Cet agent actif de décontamination peut être tout agent actif de décontamination permettant d'éliminer un contaminant quelle que soit la nature de ce contaminant : que ce contaminant soit chimique, biologique ou encore nucléaire, radioactif, ou que ce contaminant soit organique ou minéral, liquide ou solide ; ou quelle que soit la forme de ce contaminant : que ce contaminant soit sous une forme massive ou particulaire, contenu dans une couche de surface du matériau de la pièce, sous la forme d'un film ou contenu dans un film par exemple d'un film de graisses à la surface de la pièce, sous la forme d'une couche ou contenu dans une couche par exemple d'une couche de peinture à la surface de la pièce, ou tout simplement déposé sur la surface de la pièce.

Selon la nature de la contamination, les modes d'action des gels diffèrent : érosion du matériau support contenant la contamination, solubilisation de la pellicule contaminante par exemple de graisses, ou de recouvrement par exemple de peinture, ou encore inactivation *in situ* des contaminants chimiques ou biologiques dans le cas d'espèces pathogènes (anthrax).

Le gel selon l'invention peut ainsi contenir un agent actif de décontamination biologique ou chimique ou encore nucléaire radioactive ; l'agent actif de décontamination peut aussi être un agent de dégraissage, décapage. Certains agents actifs de décontamination peuvent jouer simultanément plusieurs fonctions de décontamination.

Par agent de décontamination biologique que l'on peut aussi qualifier d'agent biocide, on entend tout agent, qui lorsqu'il est mis en contact avec une espèce biologique et notamment une espèce biologique toxique est susceptible, d'inactiver ou de détruire celle-ci.

Par espèce biologique, on entend tout type de micro-organisme tel que les bactéries, les champignons, les levures, les virus, les toxines, les spores notamment les spores de Bacillus Anthracis, et les protozoaires.

Les espèces biologiques qui sont éliminées, détruites, inactivées, par le gel selon l'invention sont essentiellement des espèces bio-toxiques telles que les spores pathogènes comme par exemple les spores de Bacillus Anthracis, les toxines comme par exemple la toxine botulique, et les virus.

Par agent de décontamination chimique, on entend tout agent qui lorsqu'il est mis en contact avec une espèce chimique et notamment une espèce chimique toxique est susceptible de détruire ou d'inactiver celle-ci.

Les espèces chimiques qui sont éliminées par le gel selon l'invention sont notamment les espèces chimiques toxiques telles que les gaz toxiques, en particulier neurotoxiques ou vésicants.

Ces gaz toxiques sont notamment des composés organophosphorés, parmi lesquels on peut citer le Sarin ou agent GB, le VX, le Tabun ou agent GA, le Soman, le Cyclosarin, le diisopropyl fluoro phosphonate (DFP), l'Amiton ou agent VG, le Parathion, le Diméthoxy méthylphosphonate (DMMP). D'autres gaz toxiques sont le gaz moutarde ou agent H ou agent HD, la Lewisite ou agent L, l'agent T.

Les espèces nucléaires, radioactives qui peuvent être éliminées, par le gel selon l'invention peuvent être choisies par exemple parmi les oxydes et hydroxydes métalliques notamment sous la forme de précipités solides.

Il est à noter que dans le cas des espèces radioactives, on ne parle pas de destruction ou d'inactivation mais seulement d'élimination de la contamination par dissolution des dépôts irradiants ou corrosion des matériaux supports de la contamination. Il y a donc véritablement transfert de la contamination nucléaire vers les paillettes de gels secs.

L'agent actif de décontamination, par exemple l'agent actif de décontamination biologique ou chimique peut être choisi parmi les bases telles que l'hydroxyde de sodium, l'hydroxyde de potassium, et leurs mélanges ; les acides tels que l'acide nitrique, l'acide phosphorique, l'acide chlorhydrique, l'acide sulfurique, les hydrogénooxalates comme l'hydrogénooxalate de sodium, et leurs mélanges ; les agents oxydants tels que les peroxydes, permanganates, persulfates, l'ozone, les hypochlorites, les sels de cérium IV et leurs mélanges ; les sels d'ammonium quaternaires tels que les sels d'hexacétylpyridinium comme le chlorure d'hexacétylpyridinium ; les agents réducteurs ; et leurs mélanges.

Certains agents actifs de décontamination peuvent être classés parmi plusieurs des catégories définies plus haut.

Ainsi, l'acide nitrique est-il un acide mais aussi un agent oxydant.

Lorsque le gel contient des carraghénanes, l'agent actif de décontamination est de préférence choisi parmi l'hydroxyde de sodium, l'hydroxyde de potassium, l'hydrogénooxalate de sodium, l'acide oxalique (Exemples 8, 9 et 10) et leurs mélanges.

L'agent actif de décontamination est de préférence un agent oxydant tel qu'un peroxyde ou un sel de cérium IV, lorsque le stimulus mentionné plus haut est une variation du potentiel chimique d'oxydoréduction.

L'agent actif de décontamination, tel qu'un agent biocide, est généralement utilisé à une concentration de 0,5 à 10 mol/L de gel, de préférence de 1 à 10 mol/L, et de préférence encore de 3 à 6 mol/L de gel afin de garantir un pouvoir de décontamination, par exemple un pouvoir d'inhibition des espèces biologiques, notamment biotoxiques, compatible avec le temps de séchage du gel, et pour assurer par exemple un séchage du gel à une température comprise entre 20°C et 50°C et à une humidité relative comprise entre 20% et 60 % en moyenne en 30 minutes à 5 heures.

De manière à atteindre l'efficacité totale, y compris dans les conditions de température et d'humidité les plus défavorables vis-à-vis du temps de séchage, la formulation du gel de la présente invention supporte différentes concentrations en agent actif. On peut remarquer, en effet, que l'augmentation de la concentration en agent de décontamination plus particulièrement en agent de décontamination acide ou basique ou acide accroît considérablement la durée de séchage du gel et donc l'efficacité du procédé (Exemple 2). Pour tenir compte des cinétiques d'érosion des matériaux et des cinétiques d'inhibition des espèces contaminantes, notamment des espèces biotoxiques, l'agent actif de décontamination, notamment l'agent de décontamination biologique sera de préférence présent dans le gel à une concentration comprise entre 3 et 6 mol/l afin d'atteindre l'efficacité maximale du procédé.

L'agent actif de décontamination peut être un acide ou un mélange d'acides. Ces acides sont généralement choisis parmi les acides minéraux tels que l'acide chlorhydrique, l'acide nitrique, l'acide sulfurique et l'acide phosphorique.

Un agent décontaminant, notamment un agent décontaminant biologique particulièrement préféré est l'acide nitrique.

En effet, il s'est avéré de manière totalement surprenante que l'acide nitrique détruisait, inactivait, les espèces biologiques notamment biotoxiques.

En particulier, il a été mis en évidence de manière étonnante que l'acide nitrique assurait la destruction, l'inactivation, des spores telles que les spores de Bacillus thuringiensis qui sont des espèces particulièrement résistantes.

L'acide ou les acides est (sont) de préférence présent(s) à une concentration de 0,5 à 10 mol/L, de préférence encore de 1 à 10 mol/L, mieux de 3 à 6 mol/L pour assurer un séchage du gel généralement à une température comprise entre 20°C et 50°C et à une humidité relative comprise entre 20% et 60% en moyenne en 30 minutes à 5 heures.

Pour ce type de gel acide, l'agent viscosant inorganique est de préférence la silice ou un mélange de silices.

Ou bien, l'agent actif de décontamination par exemple l'agent actif de décontamination biologique peut être une base de préférence une base minérale, choisie de préférence parmi la soude, la potasse, et leurs mélanges.

Dans le cas d'une telle formulation de gel basique, le gel selon l'invention a, outre l'action de décontamination, une action de dégraissage.

Comme on l'a déjà mentionné plus haut, de manière à atteindre une efficacité totale, y compris dans les conditions climatiques les plus défavorables vis-à-vis du temps de séchage du gel, le gel selon l'invention peut présenter une large gamme de concentration en agent(s) de décontamination basique(s).

En effet, l'augmentation de la concentration en agent de décontamination basique comme NaOH ou KOH, jouant généralement le rôle d'agent biocide, permet d'accroître considérablement les vitesses d'inhibition des espèces biologiques, comme cela a été démontré pour des spores de Bacillus thuringiensis (Exemple 1).

La base est avantageusement présente à une concentration inférieure à 10 mol/L, de préférence entre 0,5 et 7 mol/L, de préférence encore entre 1 et 5 mol/L, mieux entre 3 et 6 mol/L, pour assurer un séchage du gel à une température comprise entre 20°C et 50°C et à une humidité relative comprise entre 20% et 60% en moyenne en 30 minutes à 5 heures.

Pour ce type de gel alcalin, basique, l'agent viscosant inorganique est de préférence une alumine ou un mélange d'alumines.

De manière à aboutir à l'efficacité maximale sur une large gamme de matériaux tout en garantissant l'innocuité du traitement, l'agent de décontamination en particulier lorsqu'il s'agit d'un agent de décontamination biologique est de préférence l'hydroxyde de sodium ou l'hydroxyde de potassium.

Dans le cas du traitement d'une matrice cimentaire, le pH basique du gel, qui est induit par l'utilisation de la soude ou de la potasse, permet d'éviter les réactions acido-basiques, entre le matériau à décontaminer et le gel, qui nuisent à l'intégrité du gel sur la surface et donc à l'efficacité du procédé.

Le caractère hygroscopique de l'hydroxyde de sodium ou de l'hydroxyde de potassium constitue également un atout considérable pour ralentir le phénomène de séchage du gel. Le temps de contact entre le gel selon l'invention, contenant par exemple une solution biocide, et la contamination biologique, s'en retrouve alors considérablement augmenté.

En effet, la compétition entre le processus d'évaporation de la phase aqueuse et celui de reprise d'eau des cristaux d'hydroxyde de sodium ou d'hydroxyde de potassium modifie favorablement la cinétique de séchage du gel (Exemple 2).

Au regard de la cinétique d'inhibition des spores (Exemple 1) et des durées de séchage des gels en fonction de la température, l'agent actif de décontamination, notamment lorsqu'il s'agit d'un agent biocide sera de préférence l'hydroxyde de sodium à une concentration comprise entre 1 et 5 mol/L.

Avantageusement, le gel selon l'invention peut contenir en outre un polymère super-absorbant.

Par « polymère super-absorbant » également dénommé « SAP », on entend généralement un polymère capable, à l'état sec, d'absorber spontanément au moins 10 fois, de préférence au moins 20 fois son poids de liquide aqueux, en particulier d'eau et notamment d'eau distillée.

Certains « SAP » peuvent absorber jusqu'à et même plus de 1000 fois leur poids de liquide.

De tels polymères super-absorbants sont notamment décrits dans l'ouvrage « Absorbent Polymer Technology, Studies in Polymer Science 8 » de L. BRANNON-PAPPAS et R. HARLAND, édition Elsevier, 1990, auquel on pourra se référer.

Par absorption spontanée, on entend un temps d'absorption allant jusqu'à environ une heure.

Le polymère super-absorbant peut avoir une capacité d'absorption d'eau allant de 10 à 2000 fois son propre poids, de préférence de 20 à 2000 fois son propre poids (soit 20 g à 2000 g d'eau absorbée par gramme de polymère absorbant), de préférence encore de 30 à 1500 fois, et en particulier de 50 à 1000 fois.

Ces caractéristiques d'absorption d'eau s'entendent dans les conditions normales de température (25°C) et de pression (760 mm Hg soit 100000 Pa) et pour de l'eau distillée.

Le SAP du gel de décontamination selon l'invention peut être choisi parmi les poly (méth) acrylates de sodium, les amidons greffés par un polymère (méth) acrylique, les amidons hydrolysés greffés par un polymère (méth) acrylique ; les polymères à base d'amidon, de gomme, et de dérivé cellulosique ; et leurs mélanges.

Plus précisément, le SAP utilisable dans le gel selon l'invention peut être par exemple choisi parmi :
- les polymères résultants de la polymérisation avec réticulation partielle de monomères à insaturation éthylénique hydrosolubles, tels que les polymères acryliques, méthacryliques (issus notamment de la polymérisation de l'acide acrylique et/ou méthacrylique et/ou de monomères acrylate et/ou méthacrylate) ou vinyliques, en particulier les poly(méth)acrylates réticulés et neutralisés, notamment sous forme de gel ; et les sels notamment les sels alcalins tels que les sels de sodium ou de potassium de ces polymères ;
- les amidons greffés par des polyacrylates ;
- les copolymères acrylamide/acide acrylique, notamment sous forme de sels de sodium ou de potassium ;
- les amidons greffés acrylamide/acide acrylique, notamment sous forme de sels de sodium ou de potassium ;
- les sels de sodium ou de potassium de carboxyméthylcellulose ;
- les sels notamment les sels alcalins, d'acides polyaspartiques réticulés ;
- les sels notamment les sels alcalins, d'acides polyglutamiques réticulés.

En particulier, on peut utiliser comme « SAP » un composé choisi parmi :
- les polyacrylates de sodium ou de potassium réticulés vendus sous les dénominations SALSORB CL 10, SALSORB CL 20, FSA type 101, FSA type 102 (Allied Colloids) ; ARASORB S-310 (Arakawa Chemical) ; ASAP 2000, Aridall 1460 (Chemdal) ; KI-GEL 201-K (Siber Hegner) ; AQUALIC CA W3, AQUALIC CA W7, AQUALIC CA W10; (Nippon Shokuba) ; AQUA KEEP D 50, AQUA KEEP D 60, AQUA KEEP D 65, AQUA KEEP S 30, AQUA KEEP S 35, AQUA KEEP S 45, AQUA KEEP Al M1, AQUA KEEP AI M3, AQUA KEEP HP 200, NORSOCRYL S 35, NORSOCRYL FX 007 (Arkema) ; AQUA KEEP 10SH-NF, AQUA KEEP J-550 (Kobo); LUQUASORB CF, LUQUASORB MA 1110, LUQUASORB MR 1600, HYSORB C3746-5 (BASF) ; COVAGEL (Sensient technologies), SANWET IM-5000D (Hoechst Celanese) ;
- les polyacrylates greffés d'amidon vendus sous les dénominations SANWET IM-100, SANWET IM-3900, SANWET IM-5000S (Hoechst) ;
- les copolymères acrylamide/acide acrylique greffés d'amidon sous forme de sel de sodium ou de potassium vendus sous les dénominations WATERLOCK A- 100, WATERLOCK A-200, WATERLOCK C-200, WATERLOCK D-200, WATERLOCK B-204 (Grain Processing Corporation) ;
- les copolymères acrylamide/acide acrylique sous forme de sel de sodium vendus sous la dénomination WATERLOCK G-400 (Grain Processing Corporation) ;
- la carboxyméthylcellulose vendue sous la dénomination AQUASORB A250 (Aqualon) ;
- le polyglutamate de sodium réticulé vendu sous la dénomination GELPROTEIN (Idemitsu Technofine).

Les polymères super-absorbants, en particulier les polymères super-absorbants (polyélectrolytes) qui contiennent des ions alcalins tels que des ions sodium ou potassium, par exemple de type poly (méth) acrylate de sodium ou de potassium, confèrent de nombreuses propriétés aux gels de décontamination selon l'invention.

Ils influencent tout d'abord la rhéologie du produit, notamment son seuil d'écoulement. En termes de mise en œuvre du procédé, l'intérêt des polymères super-absorbants est de garantir une tenue parfaite du gel sur les matériaux traités, notamment sur les surfaces verticales et en surplomb lorsque l'épaisseur de gel pulvérisé est supérieure à 1 mm.

Dans le cadre d'un procédé de décontamination notamment de décontamination biologique par gel, le polymère super-absorbant est particulièrement intéressant car il absorbe par liaison hydrogène une partie de la solution, par exemple de la solution biocide contenue dans le gel. Le nombre de liaisons hydrogènes formées entre la solution, par exemple la solution biocide, du gel et le polymère super-absorbant tel que le polyacrylate de sodium étant fonction de la charge saline, des phénomènes d'absorption/désorption apparaissent lorsque la charge saline du gel de décontamination est modifiée.

Ce mécanisme est alors particulièrement intéressant lorsqu'il s'agit de décontaminer des matériaux minéraux et poreux comme les matrices cimentaires par exemple.

En effet, au contact du matériau, la charge saline du gel augmente du fait de la présence de particules minérales très souvent à base de calcium. Au sein du polymère super-absorbant tel que le polyacrylate de sodium, la substitution du contre-ion Na⁺ par Ca²⁺ issu du calcium génère instantanément un phénomène de re-larguage de solution, par exemple de solution biocide, en raison de l'encombrement stérique plus important de l'ion calcium.

La quantité de solution, par exemple de solution biocide libérée par le polymère super-absorbant tel que le polyacrylate de sodium peut alors diffuser instantanément dans la porosité du matériau et pénétrer en profondeur.

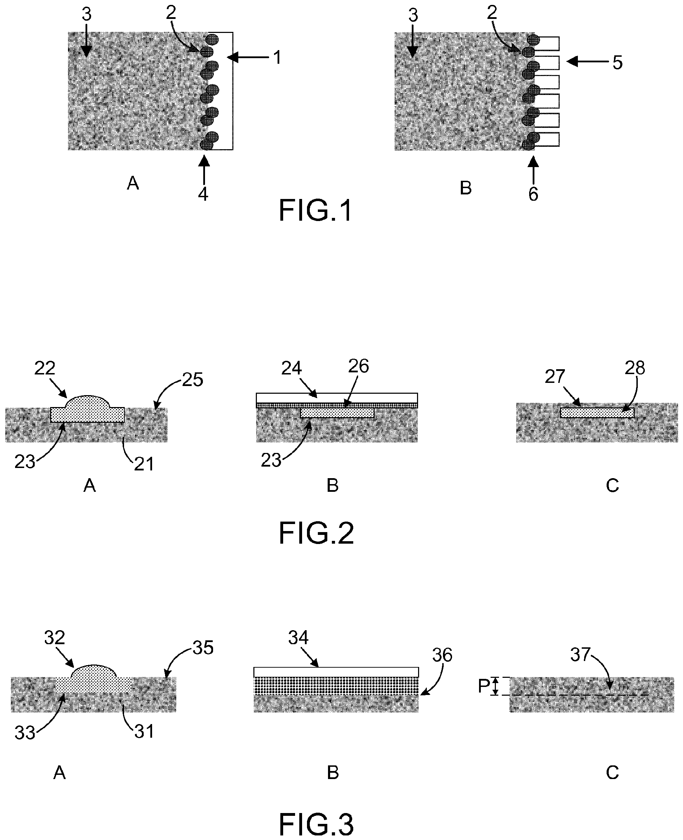

Le phénomène de diffusion de l'agent de décontamination, par exemple de l'agent biocide vers le cœur du matériau est beaucoup plus limité dans le cas d'un gel ne contenant pas de super-absorbant (voir Figures 2 et 3).

L'ajout de polymère super-absorbant au gel selon l'invention permet donc d'accroître significativement l'efficacité du gel et du procédé selon l'invention en présence de matériaux poreux contaminés en profondeur sur une épaisseur de un à plusieurs millimètres, par exemple jusqu'à 2, 5, 10, 20 voire 100 mm (Exemple 3).

Le polymère super-absorbant peut être choisi de préférence parmi les gammes Aquakeep^{®} ou Norsocryl^{®} commercialisées par la société ARKEMA.

Le gel peut aussi contenir un agent tensio-actif ou un mélange d'agents tensio-actifs, de préférence choisis parmi la famille des agents tensio-actifs non ioniques tels que les copolymères blocs, séquencés comme les copolymères séquencés d'oxyde d'éthylène et d'oxyde de propylène, et les acides gras éthoxylés ; et leurs mélanges.

Pour ce type de gel, les agents tensio-actifs sont de préférence des copolymères blocs commercialisés par la société BASF sous la dénomination "PLURONIC^{®}".

Les Pluronics^{®} sont des copolymères séquencés d'oxyde d'éthylène et d'oxyde de propylène.

Ces agents tensio-actifs influencent les propriétés rhéologiques du gel, notamment le caractère thixotropique du produit et son temps de reprise et évitent l'apparition de coulure.

Les agents tensio-actifs permettent, par ailleurs, de maîtriser l'adhésion du déchet sec (Exemple 4) et de contrôler la taille des paillettes de résidu sec pour garantir la non-pulvérulence du déchet (Exemple 5).

Le solvant selon l'invention est généralement choisi parmi l'eau, les solvants organiques, et leurs mélanges.

Un solvant préféré est l'eau, et dans ce cas, le solvant est donc constitué par de l'eau, comprend 100% d'eau.

L'invention concerne, en outre, un procédé de décontamination d'au moins une surface d'une pièce en un matériau solide, ladite surface étant contaminée par au moins une espèce contaminante se trouvant sur ladite surface et éventuellement sous ladite surface dans la profondeur de la pièce, dans lequel on réalise au moins un cycle comprenant les étapes successives suivantes :
a) on immerge la pièce dans un bain du gel tel que décrit plus haut à l'état liquide avec une viscosité inférieure à 0,1 Pa.s ;
b) on retire la pièce, revêtue d'un film du gel, du bain et on la soumet immédiatement à au moins un stimulus qui provoque une augmentation de la viscosité du gel en une durée inférieure à 5 secondes et son passage à un état gélifié avec une viscosité supérieure à 100 Pa.s ;
c) on maintient le gel sur la surface contaminée au moins pendant une durée suffisante pour que le gel détruise et/ou inactive et/ou absorbe l'espèce contaminante, et pour que le gel sèche et forme un résidu sec et solide contenant ladite espèce contaminante ;
d) on élimine le résidu sec et solide contenant ladite espèce contaminante.

Lors de l'étape b), l'augmentation rapide de la viscosité est généralement due principalement voire uniquement à l'action du stimulus sur l'agent de variation de la viscosité.

Généralement, le stimulus n'agit pas ou peu sur les autres composants du gel, tels que le solvant et ne provoque généralement pas l'élimination de ce dernier, et cette augmentation rapide de la viscosité n'est donc généralement pas due à une élimination du solvant.

Ledit stimulus a déjà été décrit en détail plus haut et peut être choisi parmi les stimuli chimiques tels qu'une variation du potentiel d'oxydoréduction du gel ; thermiques tels qu'une variation de la température du gel ; physiques tels qu'une exposition à une onde électromagnétique ; et les combinaisons de deux ou plus desdits stimuli.

Si le stimulus est une augmentation de la température du gel, il agit généralement, principalement, voire uniquement, sur l'agent de variation de la viscosité et occasionne en conséquence une augmentation rapide de la viscosité du gel, due principalement, voire uniquement, à cette action du stimulus sur l'agent de variation de la viscosité. Ce stimulus n'agit généralement pas sur le solvant et n'en provoque généralement pas l'élimination. L'augmentation rapide de la viscosité lors de l'étape b) n'est donc généralement pas due à une élimination du solvant telle que celle réalisée lors de l'étape de séchage.

Avantageusement, ledit stimulus est une diminution de la température, provoquée par exemple par le passage de la pièce d'un bain chauffé, par exemple à une température de 30°C à 80°C à l'atmosphère ambiante, par exemple à une température de 15°C à 25 °C ; ou bien ledit stimulus est le début de l'exposition du gel à un champ magnétique ou l'arrêt de l'exposition du gel à un champ magnétique.

De ce fait, le champ magnétique peut être appliqué au niveau de la cuve contenant le gel liquide ou au contraire autour de la pièce qui vient d'être nappée.

L'augmentation de la viscosité du gel lors de l'étape b) se produit en une durée inférieure à 5 secondes, de préférence inférieure à 2 secondes, de préférence encore inférieure ou égale à 1 seconde, par exemple en une durée comprise entre 0,5 seconde et 1 seconde.

Il est à noter que l'augmentation rapide de la viscosité du gel et son passage d'un état liquide à un état gélifié qui se produit lors de l'étape b) ne doit pas être confondue avec le séchage qui se produit lors de l'étape c).

En effet, le passage d'un état liquide à un état gélifié du gel lors de l'étape b) se produit généralement sans que le solvant soit éliminé. En d'autres termes, les contenus en solvant du gel à l'état liquide et du gel à l'état gélifié sont généralement sensiblement identiques même si le stimulus est une augmentation de température. Au contraire, lors du séchage réalisé lors de l'étape c), le solvant est éliminé pour former un résidu sec et solide qui n'est plus un gel à l'état liquide ou gélifié.

Avantageusement, la pièce est une pièce de petite taille et/ou de forme complexe.

Il est à noter que, dans le cas d'une surface non poreuse, la contamination par exemple la contamination biologique « inactivée » est récupérée par les paillettes de gel sec.

Par contre, dans le cas d'une contamination profonde, comme c'est le cas dans les matériaux poreux tels que les matrices cimentaires, le gel sec ne contiendra que le résidu de contamination surfacique.

La contamination interne, profonde, « inactivée » *in situ* suite à l'action du superabsorbant qui est alors avantageusement incorporé dans le gel, restera au cœur du matériau, substrat.

Avantageusement, le substrat solide est un substrat poreux, de préférence un substrat minéral poreux et le gel selon l'invention contient alors avantageusement, un polymère superabsorbant.

Toutefois, l'efficacité du gel et du procédé selon l'invention est tout aussi bonne en présence d'une surface non poreuse et/ou non minérale.

Avantageusement, le substrat est en au moins un matériau choisi parmi les métaux et les alliages métalliques comme l'acier inoxydable, l'aluminium, le plomb ; les polymères tels que les matières plastiques ou caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments et matériaux cimentaires ; les mortiers et bétons ; les plâtres ; les briques ; la pierre naturelle ou artificielle ; les céramiques.

Avantageusement, l'espèce contaminante est choisie parmi les espèces contaminantes chimiques, biologiques, nucléaires ou radioactives déjà énumérées plus haut et notamment parmi les espèces biologiques toxiques déjà énumérées plus haut.

Avantageusement, le gel est appliqué sur la surface à décontaminer à raison de 100 g à 2000 g de gel par m² de surface, de préférence de 500 à 1500 g de gel par m² de surface, de préférence encore de 600 à 1000 g par m² de surface, ce qui correspond généralement à une épaisseur de gel déposé sur la surface comprise entre 0,5 mm et 2 mm.

Avantageusement (lors de l'étape c)), le séchage est réalisé à une température de 1°C à 50°C, de préférence de 15°C à 25°C, et sous une humidité relative de 20% à 80%, de préférence de 20% à 70%.

Avantageusement, le gel est maintenu sur la surface pendant une durée de 2 à 72 heures, de préférence de 2 à 48 heures, de préférence encore de 5 à 24 heures.

Avantageusement, le résidu sec et solide se présente sous la forme de particules, par exemple de paillettes, d'une taille de 1 à 10 mm, de préférence de 2 à 5 mm.

Avantageusement, le résidu sec et solide est éliminé de la surface solide par brossage et/ou aspiration.

Avantageusement, le cycle décrit plus haut peut être répété par exemple de 1 à 10 fois en utilisant le même gel lors de tous les cycles ou en utilisant des gels différents lors d'un ou de plusieurs cycle(s).

Avantageusement, lors de l'étape c), le gel, avant séchage total, est remouillé avec une solution d'un agent de décontamination, de préférence avec une solution de l'agent actif de décontamination du gel du bain de l'étape a) dans le solvant de ce gel ce qui évite alors généralement de répéter l'application du gel sur la surface et occasionne une économie de réactif et une quantité de déchet limitée. Cette opération de remouillage peut être répétée par exemple de 1 à 10 fois.

Le procédé selon l'invention possède toutes les propriétés avantageuses inhérentes au gel de décontamination qu'il met en œuvre et qui ont déjà été largement exposées plus haut.

Le procédé selon l'invention met en œuvre pour la première fois un gel dans un procédé de décontamination par trempage.

Le procédé selon l'invention présente tous les avantages des procédés qui mettent en œuvre un gel et notamment un gel aspirable tel que celui décrit dans les documents [9] et [10] mais ne présente pas les inconvénients de ces procédés car l'application du gel est, dans le procédé selon l'invention, réalisé par trempage, immersion dans le gel à l'état liquide ce qui permet pour la première fois de traiter et de décontaminer des pièces de petite taille et/ou de forme, géométrie complexe.

De la même manière, le procédé selon l'invention présente les avantages des procédés de décontamination par trempage, immersion mais sans en présenter les inconvénients. En effet, dans le procédé selon l'invention, dès que la pièce nappée d'un film du gel liquide est sortie du bain, ledit gel prend une forme gélifiée sous l'action du stimulus, tous les risques de coulure, d'épandage et de dissémination des contaminants sont évités.

Par rapport aux procédés de décontamination par trempage dans des solutions liquides (qui ne présentent pas de transition entre une phase liquide et une phase gélifiée sous l'action d'un stimulus), le fait que le produit de la décontamination se trouve, selon l'invention, sous une forme gélifiée constitue un réel avantage pour réduire de façon significative le volume de déchets fortement contaminés.

En effet, une fois l'opération de nappage de la pièce par le gel à l'état liquide effectuée -par trempage très bref dans le bain-, la phase de décontamination proprement dite -c'est-à-dire la phase pendant laquelle le film de gel à l'état gélifié déposé va interagir avec la surface contaminée- est réalisée à l'extérieur du bain évitant ainsi la contamination de celui-ci.

Au terme d'une phase de séchage identique à celle du procédé dit « procédé gel aspirable » des documents [9] et [10], la contamination se retrouve piégée dans une quantité très limitée -par exemple inférieure à 200 g/m² de surface traitée- de paillettes de gel sec qui peuvent être facilement éliminées.

En résumé, le procédé et le gel selon l'invention présentent entre autres les propriétés avantageuses suivantes :
- l'application du gel par trempage des pièces dans un bain de gel sous forme liquide peu visqueuse (nappage), moyennant quoi le gel s'infiltre et recouvre toutes les surfaces des pièces même lorsque ces pièces sont de petite taille et/ou de géométries complexes ;
- l'adhérence aux parois de la pièce dès la sortie du bain ;
- l'obtention de l'efficacité maximale de décontamination à l'issue de la phase de séchage du gel, y compris en situation de contamination pénétrante notamment dans le cas de surfaces poreuses, auquel cas le gel contient avantageusement un polymère superabsorbant;

En général, on fait en sorte que le temps de séchage soit supérieur ou égal à la durée nécessaire pour l'inactivation des espèces contaminantes. Dans le cas d'une inactivation profonde, on fait généralement appel à un remouillage.
- le traitement d'une gamme très large de matériaux ;
- l'absence d'altération mécanique ou physique des matériaux à l'issue du traitement,
- la mise en œuvre du procédé dans des conditions climatiques variables,
- la réduction du volume de déchet,
- la facilité de récupération du déchet sec.

D'autres caractéristiques et avantages de l'invention apparaîtront mieux à la lecture de la description détaillée qui suit, cette description étant faite à titre illustratif et non limitatif, en liaison avec les dessins joints.

### BRÈVE DESCRIPTION DES DESSINS.

- La Figure 1 (A, B) présente des vues en coupe schématique illustrant les étapes principales du procédé selon l'invention pour la décontamination d'un matériau solide.
- La Figure 2 (A, B, C) présente des vues en coupe schématique montrant le mode d'action d'un gel exempt de polymère super-absorbant sur un matériau cimentaire contaminé en profondeur par une contamination sous forme liquide.
- La Figure 3 (A, B, C) présente des vues en coupe schématique montrant le mode d'action d'un gel contenant un polymère super-absorbant sur un matériau cimentaire contaminé en profondeur par une contamination sous forme liquide.
- La Figure 4 (A, B) présente les structures des carraghénanes κ (A) et 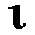 (B).
- La Figure 5 présente les conformations que prennent des chaînes de carraghénanes en fonction de la température et de la charge ionique dans le milieu.
- La Figure 6 est un schéma d'un procédé permettant d'obtenir des particules de silice ferromagnétique qui comprennent un cœur ou noyau en oxyde de fer magnétique et une coquille en silice.
- La Figure 7, qui illustre l'exemple 1, est un graphique qui représente la cinétique d'inhibition des spores de Bacillus thuringiensis, dans différentes solutions biocides liquides contenant différentes bases à diverses concentrations, à savoir : NaOH à 0,5 M, NaOH à 1M, NaOH à 5M, KOH à 0,5M, KOH à 1M, et KOH à 5M. Le nombre de spores résiduelles est donné pour chacune des solutions biocides à des temps de contact de 1 heure, 2 heures, 3 heures, 4 heures et 5 heures.
- La Figure 8, qui illustre l'exemple 2, est un graphique qui représente l'influence de la concentration en hydroxyde de sodium dans le gel sur la durée de séchage.

En ordonnée est portée la perte de masse du gel en % et en abscisse est porté le temps de séchage du gel en jours.

Les courbes A, B, C, et D représentent respectivement le séchage de gel sans NaOH (uniquement de l'eau), et avec des concentrations en NaOH de 1M, 5M et 10M.
- - La Figure 9, qui illustre l'exemple 3, est un graphique qui représente l'influence du polymère super-absorbant sur l'efficacité de la décontamination biologique d'un mortier exprimée par le nombre de spores de Bacillus thuringiensis sur un échantillon de mortier.

Pour chaque gel, les barres de gauche (en gris clair A et B) représentent la contamination des échantillons de mortier avant traitement, et les barres de droite (en gris foncé C et D) représentent la contamination résiduelle des échantillons de mortier après la récupération du gel sec.

Le graphique présente deux traitements par gel distincts, le premier (partie gauche du graphique, barres A et C accolées à gauche du graphique) en présence d'un gel biocide exempt de polymère super-absorbant, le deuxième (partie droite du graphique, barres B et D accolées à droite du graphique) en présence du même gel biocide auquel a été rajouté le polymère super-absorbant.

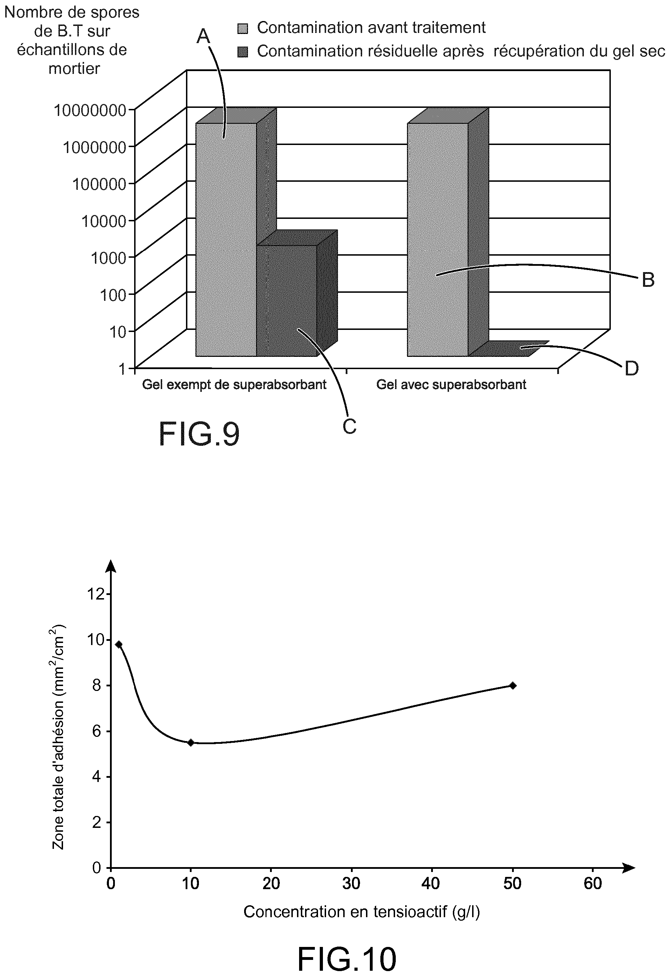

- La Figure 10, qui illustre l'exemple 4, est un graphique qui représente l'influence de la concentration en tensio-actif (Pluronic^{®}) sur le pouvoir d'adhésion des paillettes de gel sec.

En ordonnée est portée la zone totale d'adhésion (mm²/cm²), et en abscisse est portée la concentration en tensio-actif (g/L).

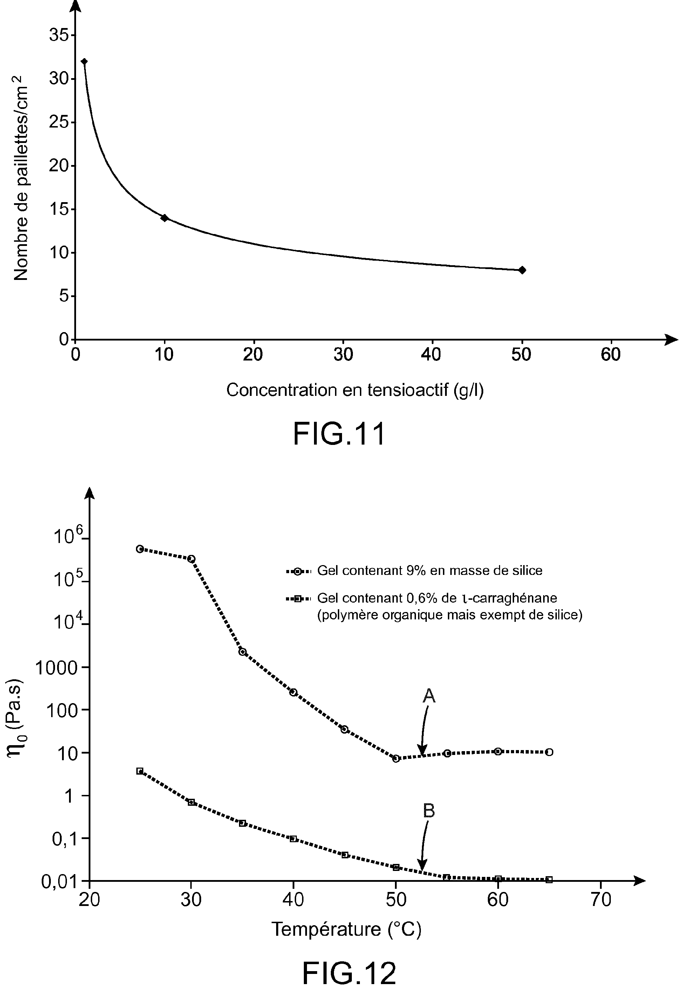

- La Figure 11, qui illustre l'exemple 5, est un graphique qui représente l'influence de la concentration en tensio-actif (Pluronic^{®}) sur le nombre de paillettes de gel sec formées.

En ordonnée est porté le nombre de paillettes/cm², et en abscisse est portée la concentration en tensio-actif (g/L).
- La Figure 12, qui illustre l'exemple 6, est un graphique qui donne l'évolution de la viscosité de deux gels en fonction de la température.

Le premier gel dont la viscosité est représentée par la courbe A est un gel aqueux qui contient 9% en masse de silice mais qui ne contient pas de carraghénane.

Le second gel dont la viscosité est représentée par la courbe B est un gel aqueux qui contient 0, 6% en masse de 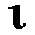 - Carraghénane mais qui ne contient pas de silice.

En ordonnée est portée la viscosité η₀ (en Pa.s), et en abscisse est portée la température T (en °C).

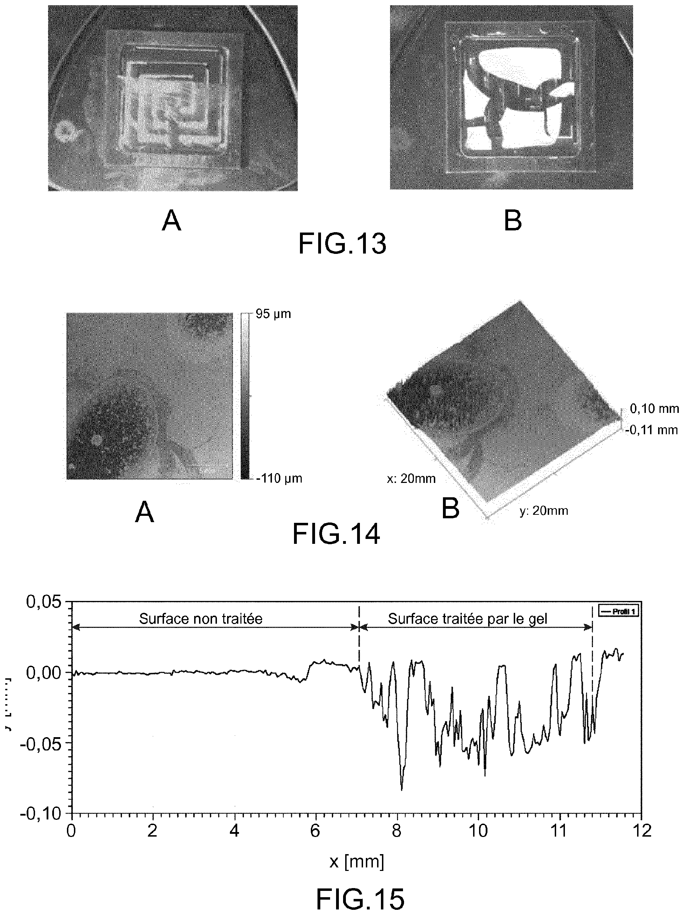

- La Figure 13 (A, B), qui illustre l'exemple 7, présente deux photographies qui montrent l'influence de la silice sur la morphologie d'un gel après séchage.

La figure (A) montre la morphologie après séchage d'un gel aqueux contenant 1,2% en masse de 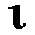 - Carraghénane mais pas de silice, et la figure (B) montre la morphologie après séchage d'un gel aqueux contenant 1,2% en masse de 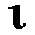 - Carraghénane et 9% en masse de silice.
- La Figure 14 (A, B), qui illustre l'exemple 8, présente deux photographies qui montrent l'érosion générée par un gel de nappage selon l'invention comprenant de l'hydrogénooxalate de sodium saturé et 1,4% en masse de κ- Carraghénane appliqué sur une plaque d'acier noir.
- La Figure 15, qui illustre l'exemple 9, est un graphique qui donne l'épaisseur (en mm) d'acier noir dégradée par un gel de nappage selon l'invention comprenant de l'hydrogénooxalate de sodium saturé et 1,4% en masse de κ-Carraghénane appliqué sur une plaque d'acier noir.

La mesure est réalisée à l'aide d'un profilomètre optique.

### EXPOSÉ DÉTAILLÉ DE MODES DE RÉALISATION PARTICULIERS

Le gel selon l'invention peut être facilement préparé généralement à la température ambiante.

Par exemple, le gel selon l'invention peut être préparé en ajoutant, de préférence progressivement, le ou les agent(s) viscosant(s) inorganique(s) par exemple la ou les alumine(s) et/ou la ou les silice(s), à une solution contenant le ou les agents(s) actif(s) de décontamination, le ou les agent(s) de variation de la viscosité, et le ou les polymères(s) super-absorbants.

Ladite solution est généralement une solution tiède, c'est-à-dire qu'elle a été portée à une température généralement de 30°C à 80°C.

Cette addition peut être réalisée en versant simplement le ou les agent(s) viscosant(s) inorganique(s) dans ladite solution. Lors de l'addition du ou des agent(s) viscosant(s) inorganique(s), la solution contenant le ou les agent(s) actif(s) de décontamination, le ou les agent(s) de variation de la viscosité, et le ou les polymère(s) super-absorbant(s) est généralement maintenue sous agitation mécanique. Cette agitation peut être, par exemple, réalisée au moyen d'un agitateur mécanique équipé d'une hélice à trois pales.

La vitesse d'agitation est généralement comprise entre 600 et 800 tours/minute.

On ajoute finalement, le ou les tensioactif(s), généralement en maintenant l'agitation mécanique.

Après la fin de l'ajout du ou des tensioactif(s), l'agitation est encore poursuivie, par exemple pendant 2 à 5 minutes, de manière à obtenir un gel parfaitement homogène.

Il est bien évident que d'autres protocoles de préparation des gels selon l'invention peuvent être mis en œuvre avec une addition des composants du gel dans un ordre différent de celui mentionné plus haut.

Généralement, le gel selon l'invention lorsqu'il se trouve dans le bain et avant toute application d'un stimulus se trouve dans un état liquide, faiblement visqueux et doit donc généralement présenter une viscosité inférieure à 100 mPa.s sous un cisaillement « nul » c'est-à-dire généralement inférieur à 5s⁻¹). Le gel liquide et peu visqueux peut ainsi s'infiltrer dans tous les recoins de la pièce et atteindre toutes les surfaces des pièces contaminées, même les plus difficiles d'accès.

Le temps de reprise de la viscosité à la sortie du bain et suite à l'application d'un stimulus doit généralement être inférieur à 5 secondes, de préférence inférieur à 2 secondes, de préférence encore inférieur ou égale à 1 seconde, par exemple en compris entre 0,5 seconde et 1 seconde et la viscosité du gel sous faible cisaillement doit généralement être supérieure à 10 Pa.s pour que le gel adhère aux surfaces et ne coule pas.

Il est à noter que l'agent tensio-actif du gel selon l'invention influence favorablement et notablement les propriétés rhéologiques du gel selon l'invention. Ce tensio-actif permet notamment d'éviter les risques d'épandage ou de coulure.

Le gel selon l'invention ainsi préparé est ensuite appliqué (1) (Figure 1) sur la surface solide (2) à décontaminer d'un substrat en un matériau solide (3), en d'autres termes sur la surface (2) ayant été exposée à une contamination par exemple à une contamination biologique (4); cette contamination a déjà été décrite plus haut. En particulier, la contamination biologique (4) peut être constituée par une ou plusieurs des espèces biologiques déjà définies plus haut.

Comme on l'a déjà indiqué plus haut, l'agent actif de décontamination, par exemple l'agent actif de décontamination biologique, est choisi en fonction de l'espèce contaminante, par exemple de l'espèce biologique à éliminer, détruire, ou inactiver.

Hormis éventuellement les alliages de métaux légers de type aluminium, dans le cas où l'on met en œuvre des gels basiques ou acides, il n'existe aucune limitation quant au matériau qui constitue la surface (2) à décontaminer, en effet, le gel selon l'invention permet de traiter sans aucun endommagement, toutes sortes de matériaux même fragiles.

Le gel selon l'invention ne génère généralement aucune altération, érosion, attaque, chimique, mécanique ou physique du matériau traité. Le gel selon l'invention n'est donc en aucune manière préjudiciable à l'intégrité des matériaux traités et permet même leur réutilisation. Ainsi, des matériels sensibles tels que des équipements militaires sont préservés et pourront après leur décontamination être réutilisés, tandis que les monuments traités par le gel selon l'invention ne sont absolument pas dégradés et voient leur intégrité visuelle et structurale conservée.

Ce matériau du substrat (3) peut donc être choisi parmi par exemple les métaux et alliages comme l'acier inoxydable, l'aluminium, et le plomb ; les polymères tels que les matières plastiques ou caoutchoucs parmi lesquels on peut citer les PVC, PP, PE notamment HDPE, PMMA, PVDF, PC; les verres ; les ciments et les matériaux cimentaires ; les mortiers et bétons ; les plâtres ; les briques ; la pierre naturelle ou artificielle ; les céramiques.

Dans tous les cas quel que soit le matériau, l'efficacité de la décontamination par le gel selon l'invention est totale.

La surface traitée peut être peinte ou non peinte.

De manière particulièrement surprenante, il s'est avéré que le gel selon l'invention, lorsqu'il contenait un polymère super-absorbant était particulièrement efficace sur les matériaux poreux tels que les matrices cimentaires comme les pâtes, les mortiers et les bétons, les briques, les plâtres, ou encore la pierre naturelle ou artificielle.

En effet, la présence dans le gel selon l'invention d'un polymère super-absorbant permet une décontamination du matériau poreux sur une profondeur beaucoup plus importante qu'avec un gel équivalent sans polymère superabsorbant.

En d'autres termes, la présence d'un polymère super-absorbant dans le gel selon l'invention facilite la diffusion de l'agent actif de décontamination, par exemple de l'agent biocide dans la profondeur du matériau lorsqu'il s'agit de traiter des substrats poreux, notamment minéraux.

L'efficacité du traitement avec le gel selon l'invention est généralement totale, y compris sur les matériaux contaminés sur plusieurs millimètres de profondeur ; dans ce dernier cas un polymère super-absorbant est alors inclus dans le gel.

Il n'existe également aucune limitation quant à la forme, la géométrie et la taille de la pièce à décontaminer, le gel selon l'invention et le procédé le mettant en œuvre permettent en particulier et avantageusement le traitement de pièces de petites tailles et/ou de formes, géométries complexes.

Par pièce de petite taille, on entend généralement une pièce dont la plus grande dimension est inférieure ou égale à 2 mètres, de préférence inférieure ou égale à 1 mètre.

Par pièce présentant une forme, géométrie complexe, on entend généralement une pièce présentant par exemple des creux, angles, recoins, évidements, etc.

Le gel selon l'invention assure le traitement efficace non seulement des surfaces horizontales des pièces mais aussi des surfaces verticales ou surfaces inclinées de ces pièces, ou encore de surfaces en surplomb de ces pièces.

Le gel selon l'invention est appliqué sur la surface à traiter par simple immersion, trempage de la pièce dans un bain contenant le gel à l'état liquide, faiblement visqueux c'est-à-dire présentant généralement la viscosité précisée plus haut.

Lors de cette immersion, trempage, la pièce et notamment la ou les surface(s) à décontaminer est(sont) nappée(s) d'un film de gel à l'état liquide.

Le trempage, l'immersion est généralement d'une durée très brève, par exemple d'une durée de 1 seconde à 60 secondes.

En fait, on peut considérer qu'une fois que la pièce est complètement immergée dans le bain à l'état liquide, elle est alors immédiatement retirée du bain.

La pièce, revêtue, nappée d'un film de gel par exemple d'une épaisseur de 1 mm, sortie du bain, est soumise immédiatement à au moins un stimulus qui provoque une reprise du gel c'est-à-dire une augmentation rapide de la viscosité du gel à l'état liquide. Le gel passe donc d'un état liquide à un état véritablement gélifié c'est-à-dire avec une structure de type gel. Dans cet état gélifié, le film de gel adhère sur les surfaces de la pièce et ne coule pas.

Le temps de reprise de la viscosité suffisamment court à savoir généralement inférieur à 5 secondes, de préférence inférieur à 2 secondes, de préférence encore inférieur ou égal à 1 seconde, par exemple en compris entre 0,5 seconde et 1 seconde , des gels selon l'invention, après application du stimulus à la sortie du bain de gel liquide, permet aux gels selon l'invention d'adhérer à toutes les surfaces des pièces à décontaminer quelle que soit la disposition, conformation de ces surfaces.

On évite ainsi toute coulure et tout épandage de solutions chimiques dans l'environnement, et toute dissémination, dispersion des espèces contaminantes.

En d'autres termes, les propriétés rhéologiques particulières de la formulation, composition décontaminante selon l'invention permettent au gel, de (re)prendre, dès la sortie du bain de trempage et de façon quasi-instantanée une viscosité suffisante qui garantit l'adhésion du film de gel sur la surface et les coulures.

La quantité de gel déposée sur la surface à traiter à la sortie du bain est généralement de 100 à 2000 g/m², de préférence de 500 à 1500 g/m², de préférence encore de 600 à 1000 g/m².

La quantité de gel déposée par unité de surface et, par voie de conséquence, l'épaisseur du gel déposé sur la surface influence la vitesse de séchage.

Ainsi, lorsque le film, couche de gel a une épaisseur de 0,5 mm à 2 mm, notamment de 1 mm sur la surface à traiter, le temps de contact efficace entre le gel et les matériaux est alors équivalent à son temps de séchage, période pendant laquelle le principe actif contenu dans le gel va interagir avec la contamination.

En outre, il a été montré de manière surprenante que la quantité de gel déposée lorsqu'elle se situe dans les plages mentionnées plus haut et en particulier lorsqu'elle est supérieure à 500 g/m² et notamment dans la plage de 500 à 1500 g/m², ce qui correspond à une épaisseur minimale de gel déposée par exemple supérieure à 500 µm pour une quantité de gel déposée supérieure à 500 g/m², permettait après séchage du gel d'obtenir une fracturation du gel sous la forme de paillettes millimétriques, par exemple d'une taille de 1 à 10 mm, de préférence de 2 à 5 mm aspirables.

La quantité de gel déposée et donc l'épaisseur de gel déposé, de préférence supérieure à 500 g/m² soit 500 µm, est le paramètre fondamental qui influence la taille des résidus secs formés après séchage du gel et qui assure ainsi que des résidus secs de taille millimétrique et non des résidus pulvérulents soient formés, de tels résidus étant facilement éliminés par un procédé mécanique et de préférence par aspiration.

Cependant, il est également à noter que grâce à l'agent tensio-actif à faible concentration, généralement de 0,1% à 2% de la masse totale du gel, le séchage du gel est amélioré et conduit à un phénomène de fracturation homogène avec une taille des résidus secs mono-dispersée et une aptitude accrue des résidus secs à se détacher du support.

Le gel est ensuite maintenu sur la surface à traiter depuis la sortie du bain et pendant toute la durée nécessaire à son séchage. Au cours de cette étape de séchage dont on peut considérer qu'elle constitue la phase active du procédé selon l'invention ou phase de décontamination, le solvant contenu dans le gel, à savoir généralement l'eau contenue dans le gel s'évapore jusqu'à l'obtention d'un résidu sec et solide.

Il est à noter que dans le procédé selon l'invention, la phase de décontamination est entièrement réalisée à l'extérieur du bain de trempage et celui-ci n'est donc pas contaminé et peut servir un très grand nombre de fois.

La durée de séchage dépend de la composition du gel dans les gammes de concentrations de ses constituants données plus haut, mais aussi, comme on l'a déjà précisé, de la quantité de gel déposée par unité de surface c'est-à-dire de l'épaisseur de gel déposé.

La durée de séchage dépend aussi des conditions climatiques à savoir de la température et de l'humidité relative de l'atmosphère dans laquelle se trouve la surface solide.

Le procédé selon l'invention peut être mis en œuvre dans des conditions climatiques extrêmement larges, à savoir à une température T de 1°C à 50°C et à une humidité relative HR de 20% à 80%.

La durée de séchage du gel selon l'invention est donc généralement de 1 heure à 24 heures à une température T de 1°C à 50°C et à une humidité relative HR de 20% à 80%.

Il est à noter que la formulation du gel selon l'invention essentiellement du fait de la présence de tensio-actifs tels que les « Pluronics^{®} » assure généralement un temps de séchage qui est sensiblement équivalent au temps de contact (entre l'agent de décontamination, tel qu'un agent biocide, et les espèces contaminantes, par exemple les espèces biologiques, notamment bio-toxiques à éliminer) qui est nécessaire, requis pour inactiver et/ou absorber les espèces contaminantes polluant le matériau et/ou pour réaliser suffisamment les réactions d'érosion de surface du matériau.

En d'autres termes, la formulation du gel assure un temps de séchage qui n'est autre que le temps d'inactivation des espèces contaminantes, par exemple des espèces biologiques qui est compatible avec la cinétique d'inhibition de la contamination par exemple de la contamination biologique.

Ou bien la formulation du gel assure un temps de séchage qui n'est autre que le temps nécessaire pour que les réactions d'érosion permettent d'éliminer une couche de surface contaminée du matériau.

Dans le cas des espèces contaminantes radioactives, la contamination est éliminée par dissolution des dépôts irradiants ou par corrosion des matériaux supports de la contamination. Il y a donc véritablement transfert de la contamination nucléaire vers les paillettes de gels secs.

La surface spécifique de la charge minérale généralement utilisée qui est généralement de 50 m²/g à 300 m²/g, de préférence de 100 m²/g et la capacité d'absorption du gel selon l'invention permettent de piéger la contamination labile (surfacique) et fixée du matériau constituant la surface à traiter.

Le cas échéant, les espèces contaminantes, par exemple les espèces biologiques contaminantes sont inactivées dans la phase gélifiée. Après séchage du gel, la contamination, par exemple la contamination biologique inactivée est éliminée lors de la récupération du résidu de gel sec décrite plus bas.

A l'issue du séchage du gel, le gel se fracture de manière homogène pour donner des résidus secs solides millimétriques, par exemple d'une taille de 1 à 10 mm, de préférence de 2 à 5 mm non pulvérulents, généralement sous la forme de paillettes solides (5) (Figure 1B).

Les résidus secs peuvent contenir la ou les espèce(s) contaminante(s) inactivée(s) (6).

Les résidus secs, tels que des paillettes (5), obtenus à l'issue du séchage présentent une faible adhérence à la surface (2) du matériau décontaminé. De ce fait, les résidus secs obtenus après séchage du gel peuvent être facilement récupérés par simple brossage et/ou aspiration. Toutefois, les résidus secs peuvent aussi être évacués par jet de gaz, par exemple par jet d'air comprimé.

Ainsi, aucun rinçage liquide n'est généralement nécessaire et le procédé selon l'invention ne génère généralement aucun effluent secondaire.

On peut cependant, bien que cela ne soit pas préféré, et si on le souhaite, éliminer les résidus secs au moyen d'un jet de liquide.

Le procédé selon l'invention réalise donc ainsi tout d'abord une importante économie de réactifs chimiques par rapport à un procédé de décontamination par lavage avec une solution. Ensuite du fait qu'un déchet sous la forme d'un résidu sec directement aspirable est obtenu, une opération de rinçage avec de l'eau ou avec un liquide, généralement nécessaire à l'élimination des traces d'agents chimiques de la pièce, est généralement évitée. Il en résulte bien évidemment une diminution significative de la quantité d'effluents produits mais aussi une simplification notable en termes de filière de traitement des déchets et d'exutoire.

En raison de la composition majoritairement minérale du gel selon l'invention et de la faible quantité de déchets produits, le déchet sec peut être stocké ou dirigé vers une filière d'évacuation (« exutoire ») sans traitement préalable. Les paillettes de gel sec obtenues à l'issue du procédé selon l'invention ont été agréées auprès de l'ANDRA en tant que déchet hétérogène immobilisable dans un coulis de mortier HTC.

A l'issue du procédé selon l'invention, on récupère un déchet solide sous forme de paillettes pouvant être conditionnées en l'état, directement conditionnables, il résulte comme on l'a déjà indiqué plus haut une diminution significative de la quantité d'effluents produits ainsi qu'une simplification notable en termes de filière de traitement du déchet et d'exutoire.

De plus, dans le domaine du nucléaire, le fait de ne pas avoir à retraiter les paillettes avant le conditionnement du déchet constitue un atout considérable ; cela autorise l'utilisation d'agent actifs performants jusqu'ici interdits dans les bains liquides de décontamination en raison des contraintes d'exploitation des stations de traitements des effluents liquides (« STEL »).

Le bain de gel pourra donc contenir des agents oxydants puissants. Ces derniers pourront, si besoin, être régénérés *in situ* au moyen d'électrodes implantées dans le bain. C'est le cas du cérium IV qui peut très facilement être régénéré à partir de l'électrolyse du cérium III.

A titre d'exemple, dans le cas courant où l'on applique 1000 grammes de gel par m² de surface traitée, la masse de déchet sec produite est inférieure à 200 grammes par m².

Sur la Figure 2, on a illustré la décontamination par un gel ne contenant pas de polymère super-absorbant d'un substrat poreux (21) contaminé, par exemple par des spores en solution aqueuse (22). Le front de contamination (23) s'étend dans la profondeur du substrat (Figure 2A). Lorsque l'on applique un gel biocide (24) sur la surface (25) du substrat, le front de diffusion (26) de l'agent biocide s'étend peu dans la profondeur du substrat et reste en-deçà du front de contamination (23) (Figure 2B). De ce fait, lorsque le gel est enlevé (Figure 2C) la zone assainie (27) s'étend peu en profondeur et il reste une contamination résiduelle (28) dans le substrat poreux (21).

Sur la Figure 3, on a illustré la décontamination, par un gel contenant un polymère super-absorbant, d'un substrat poreux (31) contaminé, par exemple par des spores en solution aqueuse (32). Le front de contamination (33) s'étend dans la profondeur du substrat (Figure 3A). Lorsque l'on applique un gel biocide contenant le super-absorbant(34) sur la surface (35) du substrat, le front de diffusion (36) de l'agent biocide s'étend dans la profondeur du substrat et va au-delà du front de contamination (Figure 3B). De ce fait, la zone assainie (37) s'étend en profondeur (P) et il ne reste plus de contamination résiduelle dans le substrat poreux.

L'invention va maintenant être décrite en référence aux exemples suivants, donnés à titre illustratif et non limitatif.

### EXEMPLES :

### Exemple 1 :

Dans cet exemple, on étudie la cinétique d'inhibition des spores de Bacillus thuringiensis, dans différentes solutions biocides liquides contenant différentes bases à diverses concentrations, à savoir : NaOH à 0,5 M, NaOH à 1M ; NaOH à 5M, KOH à 0,5M, KOH à 1M, et KOH à 5M.

### Protocole expérimental :

L'expérimentation consiste à mettre en contact, sous agitation, 2x10⁶ spores avec 1 ml de solution biocide liquide.

A l'issue de 1 heure, 2 heures, 3 heures, 4 heures, et 5 heures d'agitation, des prélèvements de mélange sont effectués pour révéler l'activité biologique du mélange et déterminer la cinétique d'inhibition des spores dans le milieu biocide considéré.

La révélation consiste alors à déposer une goutte de mélange sur un milieu nutritif (Gel Agar) et à dénombrer, à l'issue d'une période d'incubation de 16 heures à 30°C, le nombre de colonies formées. Chacune des colonies étant le résultat d'une spore inactivée.

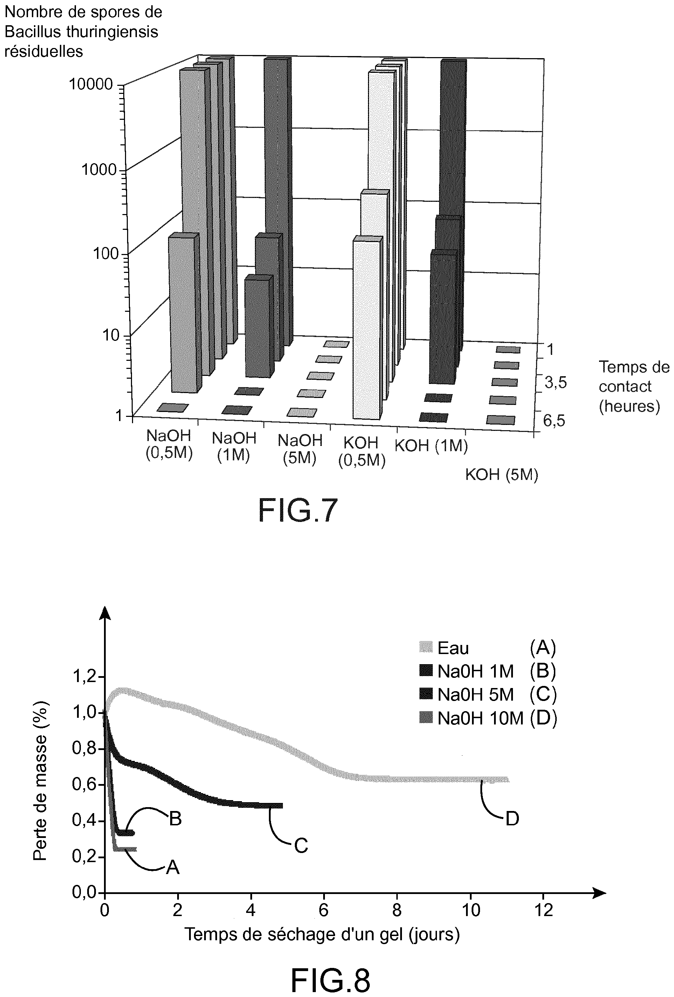

Les résultats des essais sont donnés sur la Figure 7 où le nombre de spores résiduelles est donné pour chacune des solutions biocides à des temps de contact de 1 heure, 2 heures, 3 heures, 4 heures, 5 heures.

La Figure 7 montre que l'augmentation de la concentration en agent biocide permet d'accroître considérablement les vitesses d'inhibition des spores de Bacillus thuringiensis.

### Exemple 2 :

Dans cet exemple, on étudie l'influence de la concentration en hydroxyde de sodium dans un gel (sur la durée de séchage.

Le gel a la composition suivante en pourcentages massiques :
- Alumine : 14%
- Solution d'hydroxyde de sodium (concentration variable) : 85%
- Tensio-actif (Pluronic^{®} P8020) : 0,7%
- Polymère super-absorbant : Polyacrylate de sodium Norsocryl^{®} S35 : 0,3%.

### Protocole expérimental :

Les gels, de concentration en hydroxyde de sodium variable (0 M, 1 M, 5 M et 10 M) sont étalés sur un support métallique inerte sur une épaisseur contrôlée de 1 mm. Le support métallique contenant le film de gel est alors placé dans une enceinte climatique équipée d'une balance de précision qui assure le suivi de la perte de masse du gel au cours du temps. L'enceinte climatique est régulée à une température de 22°C et à une humidité relative de 60%.

Les courbes de la Figure 8 montrent que le caractère hygroscopique de l'hydroxyde de sodium (mais aussi de l'hydroxyde de potassium) ralentit le phénomène de séchage du gel. De ce fait, le temps de contact entre l'agent de décontamination, à savoir la solution biocide, et la contamination biologique se trouve considérablement augmenté.

Cet exemple n'est pas réalisé avec un gel conforme en tous points au gel selon l'invention, cependant les résultats de cet exemple peuvent s'appliquer aussi à un gel selon l'invention.

L'effet de la concentration en soude sur le temps de séchage est le même pour le gel de cet exemple que pour un gel de la présente invention.

### Exemple 3 :

Dans cet exemple, on étudie l'influence du polymère super-absorbant sur l'efficacité de la décontamination biologique d'un mortier exprimée par le nombre de spores de Bacillus thuringiensis sur un échantillon de mortier.

### Protocole expérimental :

Les échantillons de mortier sont contaminés par dépôt d'une goutte d'eau d'un volume de 100 µl contenant 2x10⁷ spores de Bacillus thuringiensis.

Après diffusion de la solution contaminante dans la profondeur du matériau cimentaire, les gels biocides de décontamination sont étalés sur la face contaminée des échantillons de mortier. La quantité de gel déposé est égale à 1000 g/m².

A l'issue de 24 heures de séchage, les paillettes de gel sec formées sont éliminées de l'échantillon de mortier. Ce dernier est alors immergé dans une solution nutritive Luria Broth maintenue sous agitation pendant 3 heures à une température de 37°C.

La révélation de l'activité biologique résiduelle des échantillons de mortier consiste alors à prélever un volume connu de solution nutritive Luria Broth dans lequel ont trempé les échantillons de mortier et à le déposer sur un gel agarose. Après 24 heures d'incubation, le dénombrement des colonies de bactéries permet de révéler le nombre de spores non inactivées par le gel biocide de décontamination.

Le gel comprenant un polymère super-absorbant a la composition suivante en pourcentages massiques :
- Alumine : 14%
- Solution d'hydroxyde de sodium (1M) : 85%
- Tensio-actif (Pluronic^{®} P8020) : 0,7%
- Polymère super-absorbant : Polyacrylate de sodium Norsocryl^{®} S35 : 0,3%

Le gel exempt de polymère absorbant a la même composition sauf que le polymère super-absorbant est omis.

Le graphique de la Figure 9 montre que l'ajout de polymère super-absorbant permet d'accroître significativement l'efficacité de la décontamination d'un matériau poreux tel qu'un mortier qui est contaminé en profondeur sur une épaisseur de plusieurs millimètres.

Cet exemple n'est pas réalisé avec un gel conforme en tous points au gel selon l'invention, cependant les résultats de cet exemple peuvent s'appliquer aussi à un gel selon l'invention.

L'effet de l'addition d'un polymère super-absorbant sur l'efficacité de la décontamination biologique est le même pour le gel de cet exemple que pour un gel de la présente invention.

### Exemple 4 :

Dans cet exemple, on étudie l'influence de la concentration en tensio-actif, à savoir le Pluronic^{®} P 8020, sur le pouvoir d'adhésion des paillettes de gel sec.

Le gel étudié est un gel qui a la composition suivante en pourcentages massiques :
- Alumine : 14% ;
- Solution d'hydroxyde de sodium (1M) : 85% ;
dans lequel on fait varier la concentration en tensio-actif (Pluronic^{®} P8020).

Les autres composés étant maintenus à des teneurs massiques égales.

Les concentrations en tensio-actifs sont de 0 g/L ; 10 g/L ; et 50 g/L.

### Protocole expérimental :

Le gel est appliqué sur une lame d'acier inoxydable flexible (feuille calibrée de chez Outillage francilien), dont les propriétés mécaniques sont connues (25 µm d'épaisseur, longueur 2 cm, largeur 1 cm et un module de Young de 2.10¹¹ Pa), l'une des extrémités est fixe et l'autre libre. On nivelle la surface de la couche de gel avec un racleur approprié afin d'y déposer une épaisseur constante de un mm.

On ajoute deux caméras, une caméra placée au-dessus de la couche de gel permet de visualiser l'apparition des fractures et une autre placée sur le côté permet de mesurer l'évolution de l'épaisseur de la couche de gel au cours du temps. L'adhésion des paillettes est étudiée par analyses des images obtenues par les deux caméras.

Le graphique de la Figure 10 montre que dans la gamme de concentration qui nous intéresse (< 10 g/l), l'augmentation de la concentration en Pluronic^{®} engendre une diminution de l'adhésion des paillettes. La récupération du déchet de gel sec par brossage et/ou aspiration s'en retrouve alors facilitée.

Cet exemple n'est pas réalisé avec un gel conforme en tous points au gel selon l'invention, cependant les résultats de cet exemple peuvent s'appliquer aussi à un gel selon l'invention.

L'effet de la concentration en tensio-actif sur le pouvoir d'adhésion des paillettes de gel sec est le même pour le gel de cet exemple que pour un gel de la présente invention.

### Exemple 5 :

Dans cet exemple, on étudie l'influence de la concentration en tensio-actif, à savoir le Pluronic^{®} P 8020, sur le nombre de paillettes de gel sec formées.

Le gel étudié est le gel de l'exemple 4 dans lequel on fait varier la concentration en tensio-actif.

Les concentrations en tensio-actifs sont de 0 g/L;10 g/L ; et 50 g/L.

Le protocole expérimental utilisé est rigoureusement le même que celui utilisé dans l'exemple 4.

Le graphique de la Figure 11 montre que l'ajout de Pluronic^{®} à la formulation du gel engendre une diminution du nombre de paillettes. L'ajout de Pluronic^{®} permet d'améliorer la ténacité de la matrice gélifiée face à la fracturation induite par le séchage : le gel va se fracturer plus difficilement, le nombre de fractures sera moindre et le nombre de paillettes sera ainsi plus petit. En ce qui concerne le procédé de décontamination, la diminution du nombre de paillettes est bénéfique : les paillettes étant plus grosses, le déchet sera non pulvérulent lors de la phase de récupération du déchet par brossage et/ou aspiration.

Cet exemple n'est pas réalisé avec un gel conforme en tous points au gel selon l'invention, cependant les résultats de cet exemple peuvent s'appliquer aussi à un gel selon l'invention.

L'effet de la concentration en tensio-actif sur le nombre de paillettes de gel sec formées est le même pour le gel de cet exemple que pour un gel de la présente invention.

### Exemple 6.

Dans cet exemple, on étudie l'évolution de la viscosité de gels en fonction de la température.

Les deux gels étudiés ont les compositions suivantes en pourcentages massiques :
Premier gel :
   - Silice : 9%.
   - H₂O : 91%.
Second gel :
   - 1-Carraghénane : 0,6%
   - H₂O : 99,4%.

Les résultats de cette étude sont portés sur la Figure 12.

Cet exemple montre que les particules minérales de type silice jouent le rôle de viscosant pour permettre à la solution de se gélifier et d'adhérer ainsi aux surfaces et que la viscosité du gel contenant du carraghénane diminue avec la température.

### Exemple 7.

Dans cet exemple, on étudie l'influence de la silice sur la morphologie de gels après séchage.

Les deux gels étudiés ont les compositions suivantes en pourcentages massiques :
Premier gel :
   - t-Carraghénane : 1,2%
   - H₂O : 98,8%
Second gel :
   - 1-Carraghénane : 1,2%
   - Silice : 9%
   - H₂O : 89,8%

### Protocole expérimental.

Les gels sont déposés sur un échantillon d'acier inoxydable de 4 cm de côté et évidé sur une profondeur égale à 1 mm.

L'objectif est ici de maîtriser parfaitement l'épaisseur de gel déposé. Dès lors, les gels sont soumis à une phase de séchage à température ambiante (22°C) jusqu'à ce que l'évolution de la masse des gels n'évolue plus.

Les photographies des figures 13A et B montrent que les particules minérales de type silice assurent la fracturation du gel lors du séchage pour aboutir à un déchet sec sous forme de paillettes (13B) ce qui n'est pas le cas du gel qui ne contient pas de silice (13A).

### Exemple 8.

Dans cet exemple, on traite la surface d'une plaque en acier noir par un gel de nappage selon l'invention et on observe ensuite la plaque ainsi traitée.

Le gel étudié est un gel qui a la composition suivante en pourcentages massiques :
- Solution aqueuse d'hydrogénooxalate de sodium saturée: 98,6%
- -κ- Carraghénane : 1,4%

### Protocole expérimental.

L'expérimentation consiste à déposer sur une plaque en acier noir une goutte de gel préalablement chauffé à une température de 50°C. Le gel ainsi sous forme liquide est déposé sur la plaque à l'aide d'une pipette pasteur puis abandonné au séchage à température ambiante (22°C). Une fois sec, le gel est récupéré par brossage des paillettes. L'érosion de la plaque est alors caractérisée en réalisant des photographies de la plaque.

Les photographies de la Figure 14 (A et B) montrent qu'une érosion a été effectivement générée sur la plaque en acier noir par le gel de nappage.

### Exemple 9.

Dans cet exemple, on traite une partie de la surface d'une plaque en acier noir par un gel de nappage selon l'invention, tandis qu'une autre partie de cette surface ne subit pas de traitement.

Le gel étudié est un gel qui a la composition suivante en pourcentages massiques :
- Solution aqueuse d'hydrogénooxalate de sodium saturée: 98,6%
- -κ- Carraghénane : 1,4%

### Protocole expérimental.

L'expérimentation consiste à déposer sur une plaque d'acier noir une goutte de gel préalablement chauffé à une température de 50°C. Le gel ainsi sous forme liquide est déposé sur la plaque à l'aide d'une pipette pasteur puis abandonné au séchage à température ambiante (22°C). Une fois sec, le gel est récupéré par brossage des paillettes. L'érosion de la plaque est alors caractérisée par profilomètrie optique.

On réalise ensuite des mesures à l'aide d'un profilomètre optique sur la partie de la surface traitée par le gel et sur la partie de la surface non traitée par le gel (voir Figure 15).

Ces mesures montrent que la surface traitée par le gel a effectivement subi une érosion sur une profondeur pouvant atteindre jusqu'à 60 à 70 µm.

### Exemple 10 :

Des essais de décontamination à l'aide d'une formulation de type « gels de nappage » ont été réalisés. Ces expériences visent à démontrer l'efficacité d'un tel gel en situation « réelle », c'est-à-dire sur un matériau type contaminé par des radio-éléments.

Le matériau à décontaminer a été préparé comme suit : une quantité correspondant à environ 5000 Bq de ⁶⁰Co, émetteur y, a été déposée par « dépôt marguerite » sur des plaques en acier noir (1,5 x 1,5 cm environ). Ces plaques (plaques 1 et 2) ont ensuite été séchées à l'épiradiateur (séchage infrarouges). L'activité des plaques est enfin mesurée par comptage gamma (résultats donnés ci-dessous dans le tableau).

En parallèle, un « gel de nappage » a été préparé. Il s'agit d'une formulation contenant 8% de silice, (Tixosil 331) mélangée à une solution d'acide oxalique saturée et de soude (en proportions équimolaires), dans laquelle sont ajoutés à chaud 1,4g / 100 mL de κ-carraghénane.

Le matériau obtenu a comme caractéristique principale une variation importante de sa viscosité en fonction de la température, autorisant le procédé de nappage.

Le gel, solide à basse température, a été fondu et appliqué sur les pièces à décontaminer. Après quelques heures de séchage, les résidus secs formés sont éliminés et on mesure la contamination résiduelle de la même façon que précédemment par comptage gamma des plaques.

Voici les résultats obtenus sur deux plaques d'acier noir (à +/- 5%) :

| | Plaque 1 | Plaque 2 |
|---|---|---|
| Dépôt initial ⁶⁰Co | 5307 Bq | 5600 Bq |
| ⁶⁰Co après décontamination du gel | 320 Bq | 325 Bq |

Soit un facteur de décontamination de 17 environ.

Pour ces essais, il a donc été démontré l'efficacité de décontamination d'un gel de nappage à l'aide de l'acide oxalique sur de l'acier noir contaminé par un dépôt de ⁶⁰Co.

Cet exemple n'est pas réalisé avec un gel conforme en tous points au gel selon l'invention, cependant les résultats de cet exemple peuvent s'appliquer aussi à un gel selon l'invention.

### RÉFÉRENCES

[1] JENEVEIN. E, "Cleaning composition for neutralizing biological and chemical weapons removal agents", US-B2-7,026,274.
[2] SCHILLING. A, HODGE. R, "Peracid-based large area decontamination", US-A1-2006/0073067.
[3] CONERLY. L, EHNTHOLT. D, LOUIE. A, WHELAN. R, "Chemical and/or biological decontamination system", US-A1-2003/0109017.
[4] TUCKER. M, COMSTOCK. R, "Decontamination formulation with sorbent additive", US-A1-2004/0022867.
[5] ROGERS. J.V, SABOURIN. C.L.K, CHOI. Y.W, "Decontamination assessment of bacillus subtilis, and Geobacillus stearothermophilus spores on indoor surfaces using a hydrogen peroxide gas generator", 2005*.*
[6] JOSSE. D, BOUDRY. I, NAUD. N, "Décontamination cutanée vis-à-vis des agents organophosphorés et de l'ypérite au soufre : Bilan et perspectives", Médecine et armées, vol. 34, n°1, pages 33-36, 2006.
[7] HOFFMAN. D, Mc GUIRE. R, "Oxidizer gels for detoxification of chemical and biological agents", US-B1-6,455,751.
[8] HARPER. B, LARSEN. L, "A comparison of decontamination technologies for biological agents on selected commercial surface materials", Biological weapons improved response program, April 2001.
[9] FAURE. S, FOURNEL. B, FUENTES. P, LALLOT. Y., "Procédé de traitement d'une surface par un gel de traitement, et gel de traitement", FR-A1-2 827 530.
[10] FAURE. S, FUENTES. P, LALLOT. Y., "Gel aspirable pour la décontamination de surfaces et utilisation", FR-A1-2 891470.
[13] YURII. A., "Sol-gel-derived biomaterials of silica and carrageenans", Journal of Colloid and Interface Science, 268 (2003) 68-76, 2003.
[14] FISHERIES AND AQUACULTURE DEPARTMENT. "Training Manual on Gracilaria Culture and Seaweed Processing in China", Food and Agriculture Organization of the United Nations, 1990.
[15] BELLAN. C., "Etude et utilisation de matériaux adaptatifs dans le contrôle actif de vibrations", Thèse de l'Université de Nice Sophia-Antipolis, UFR Sciences, 2001.
[16] SUNG. T. L, MIN. S. C., "Magnetorheological characterization of carbonyl iron based suspension stabilized by fumed silica", Journal of Magnetism and magnetic Materials, 282, 170-173, 2004.
[17] KROELL. M, PRIDOEHL. M, ZIMMERMANN. G., "Magnetic and Rheological characterization of novel ferrofluids", Journal of Magnetism and magnetic Materials, 289, 21-24, 2005.
[18] SANG. H, THURSTON. H., "Synthesis and characterization of monodisperse silica colloids loaded with superparamagnetic iron oxide nanoparticles", Chemical Physics Letters, 401, 19-23, 2005.
[19] KUANG. G, JI. Y, JIA. X., "Photoresponsive organogels: an amino acid-based Dendron functionalized with p-nitrocinnamate", Tetrahedron, 65, 3496-3501, 2009.
[20] PIEPENBROCK. M, LLOYD. G, CLARKE. N, STEED. J., "Metal and Anion Binding Supramolecular Gels", Chem. Rev. ACS, 2009.
[21] WENG. W, BECK. B, JAMIESON. A., "Understanding the Mechanism of Gelation and Stimuli-Responsive Nature of Metallo-Supramolecular Gels", J. Am. Chem. Soc, 128, 11663-11672, 2006.

## Revendications

1. Gel de décontamination, constitué par une solution colloïdale comprenant ou constituée par :
- 0,1% à 30% en masse, de préférence 0,1% à 25% en masse, de préférence encore 5% à 25% en masse, mieux 8% à 20% en masse, par rapport à la masse du gel, d'au moins un agent viscosant inorganique ;
- 0,01% à 5% en masse, de préférence 0,05% à 3% en masse, par rapport à la masse du gel, d'au moins un agent de variation de la viscosité qui, lorsqu'il est soumis à au moins un stimulus, de préférence externe au gel, provoque une variation de la viscosité du gel,
ledit stimulus, de préférence externe au gel, étant choisi parmi les stimuli chimiques tels qu'une variation du potentiel d'oxydoréduction du gel ; thermiques tels qu'une variation de la température du gel ; physiques tels qu'une exposition à une onde électromagnétique ; et les combinaisons de deux ou plus desdits stimuli,
ledit agent de variation de la viscosité n'étant pas un polymère superabsorbant,
ledit agent de variation de la viscosité étant choisi dans le groupe constitué par les polysaccharides, les composés oxydants, les particules magnétiques et les composés dont la viscosité varie lorsqu'ils sont exposés à des radiations électromagnétiques de longueurs d'ondes différentes ;
- 0,1 à 10 mol/L de gel, de préférence 0,5 à 10 mol/L de gel, de préférence encore 1 à 10 mol/L de gel, d'au moins un agent actif de décontamination différent dudit agent de variation de la viscosité ;
- 0,1% à 2% en masse par rapport à la masse du gel, d'au moins un agent tensio-actif et de préférence d'au moins un agent tensio-actif non ionique ;
- éventuellement 0,05% à 5% en masse, de préférence 0,05% à 2% en masse par rapport à la masse du gel, d'au moins un polymère super-absorbant ;
- et le reste de solvant.

2. Gel de décontamination selon la revendication 1, dans lequel l'agent viscosant inorganique est choisi parmi les alumines, les silices, les aluminosilicates, les argiles, et leurs mélanges ; de préférence l'agent viscosant inorganique est choisi parmi les silices pyrogénées, les silices précipitées, les silices hydrophiles, les silices hydrophobes, les silices acides, les silices basiques, et leurs mélanges ; de préférence encore l'agent viscosant inorganique est constitué par un mélange d'une silice précipitée et d'une silice pyrogénée.

3. Gel de décontamination selon la revendication 2, dans lequel l'agent viscosant inorganique est constitué par une ou plusieurs alumine(s) représentant de 5% à 30% en masse, de préférence de 8% à 17% en masse par rapport à la masse du gel.

4. Gel de décontamination selon la revendication 1, dans lequel l'agent de variation de la viscosité est choisi parmi les carraghénanes.

5. Gel de décontamination selon l'une quelconque des revendications précédentes, dans lequel l'agent actif de décontamination est choisi parmi les bases telles que l'hydroxyde de sodium, l'hydroxyde de potassium, et leurs mélanges ; les acides tels que l'acide nitrique, l'acide phosphorique, l'acide chlorhydrique, l'acide sulfurique, les hydrogénooxalates comme l'hydrogénooxalate de sodium, et leurs mélanges ; les agents oxydants tels que les peroxydes, les permanganates, les persulfates, l'ozone, les hypochlorites, les sels de cérium IV, et leurs mélanges ; les sels d'ammonium quaternaires tels que les sels d'hexacétylpyridinium comme le chlorure d'hexacétylpyridinium ; les agents réducteurs; et leurs mélanges ; le polymère super-absorbant est choisi parmi les poly (méth) acrylates de sodium, les amidons greffés par un polymère (méth) acrylique, les amidons hydrolysés greffés par un polymère (méth) acrylique ; les polymères à base d'amidon, de gomme, et de dérivé cellulosique ; et leurs mélanges ; l'agent tensio-actif non ionique est choisi parmi les copolymères blocs, séquencés comme les copolymères séquencés d'oxyde d'éthylène et d'oxyde de propylène, et les acides gras éthoxylés ; et leurs mélanges ; et le solvant est choisi parmi l'eau, les solvants organiques et leurs mélanges.

6. Procédé de décontamination d'au moins une surface d'une pièce en un matériau solide, ladite surface étant contaminée par au moins une espèce contaminante se trouvant sur ladite surface et éventuellement sous ladite surface dans la profondeur de la pièce, dans lequel on réalise au moins un cycle comprenant les étapes successives suivantes :
a) on immerge la pièce dans un bain du gel selon l'une quelconque des revendications 1 à 5, à l'état liquide avec une viscosité inférieure à 0,1 Pa.s ;
b) on retire la pièce, revêtue d'un film du gel, du bain et on la soumet immédiatement à au moins un stimulus qui provoque une augmentation de la viscosité du gel en une durée inférieure à 5 secondes et son passage à un état gélifié avec une viscosité supérieure à 100 Pa.s ;
c) on maintient le gel sur la surface contaminée au moins pendant une durée suffisante pour que le gel détruise et/ou inactive et/ou absorbe l'espèce contaminante, et pour que le gel sèche et forme un résidu sec et solide contenant ladite espèce contaminante ;
d) on élimine le résidu sec et solide contenant ladite espèce contaminante.

7. Procédé selon la revendication 6, dans lequel ledit stimulus est choisi parmi les stimuli chimiques tels qu'une variation du potentiel d'oxydoréduction du gel ; thermiques tels qu'une variation de la température du gel ; physiques tels qu'une exposition à une onde électromagnétique ; et les combinaisons de deux ou plus desdits stimuli ; de préférence ledit stimulus est une diminution de la température, provoquée par exemple par le passage de la pièce d'un bain chauffé, par exemple à une température de 30°C à 80°C à l'atmosphère ambiante, par exemple à une température de 15°C à 25°C; ou bien ledit stimulus est le début de l'exposition du gel à un champ magnétique ou l'arrêt de l'exposition du gel à un champ magnétique.

8. Procédé selon la revendication 6 ou 7, dans lequel l'augmentation de la viscosité du gel lors de l'étape b) se produit en une durée inférieure à 2 secondes, de préférence inférieure ou égale à 1 seconde, par exemple en une durée comprise entre 0,5 seconde et 1 seconde.

9. Procédé selon l'une quelconque des revendications 6 à 8, dans lequel la pièce est une pièce de petite taille et/ou de forme complexe.

10. Procédé selon l'une quelconque des revendications 6 à 9, dans lequel le matériau solide est choisi parmi les métaux et les alliages métalliques comme l'acier inoxydable, l'aluminium et le plomb ; les polymères tels que les matières plastiques ou caoutchoucs comme les poly(chlorure de vinyle)s ou PVC, les polypropylènes ou PP, les polyéthylènes ou PE notamment les polyéthylènes haute densité ou HDPE, les poly(méthacrylate de méthyle)s ou PMMA, les poly(fluorure de vinylidène)s ou PVDF, les polycarbonates ou PC ; les verres ; les ciments et les matériaux cimentaires; les mortiers et bétons ; les plâtres ; les briques ; la pierre naturelle ou artificielle ; les céramiques.

11. Procédé selon l'une quelconque des revendications 6 à 10, dans lequel l'espèce contaminante est choisie parmi les espèces contaminantes chimiques, biologiques, nucléaires ou radioactives, par exemple l'espèce contaminante est une espèce biologique choisie parmi les bactéries, les champignons, les levures, les virus, les toxines, les spores et les protozoaires, en particulier l'espèce biologique est choisie parmi les espèces bio-toxiques telles que les spores pathogènes comme par exemple les spores de Bacillus anthracis, les toxines comme par exemple la toxine botulique, et les virus.

12. Procédé selon l'une quelconque des revendications 6 à 11, dans lequel le gel est appliqué sur la surface à raison de 100 g à 2000 g de gel par m² de surface, de préférence de 500 g à 1500 g de gel par m², de préférence encore de 600 g à 1000 g de gel par m² de surface.

13. Procédé selon l'une quelconque des revendications 6 à 12, dans lequel lors de l'étape c), le séchage est réalisé à une température de 1°C à 50°C, de préférence de 15°C à 25°C, et sous une humidité relative de 20% à 80%, de préférence de 20% à 70%.

14. Procédé selon l'une quelconque des revendications 6 à 13, dans lequel le gel est maintenu sur la surface pendant une durée de 2 à 72 heures, de préférence de 2 à 48 heures, de préférence encore de 5 à 24 heures.

15. Procédé selon l'une quelconque des revendications 6 à 14, dans lequel le résidu sec et solide se présente sous la forme de particules, par exemple de paillettes, d'une taille de 1 à 10 mm, de préférence de 2 à 5 mm.

16. Procédé selon l'une quelconque des revendications 6 à 15, dans lequel le résidu sec et solide est éliminé de la surface solide par brossage et/ou aspiration.

17. Procédé selon l'une quelconque des revendications 6 à 16, dans lequel le cycle décrit est répété de 1 à 10 fois en utilisant le même gel lors de tous les cycles ou en utilisant des gels différents lors d'un ou de plusieurs cycle(s).

18. Procédé selon l'une quelconque des revendications 6 à 17, dans lequel lors de l'étape c), le gel, avant séchage total, est remouillé avec une solution d'un agent de décontamination, de préférence avec une solution de l'agent actif de décontamination du gel du bain de l'étape a) dans le solvant de ce gel.

## Patentansprüche

1. Dekontaminationsgel, bestehend aus einer kolloidalen Lösung mit oder bestehend aus:
- 0,1 bis 30 Gew.-%, vorzugsweise 0,1 bis 25 Gew.-%, vorzugsweise noch 5 bis 25 Gew.-%, besonders bevorzugt 8 bis 20 Gew.-%, bezogen auf die Masse des Gels, mindestens eines anorganischen Viskositätsmittels;
- 0,01 bis 5 Gew.-%, vorzugsweise 0,05 bis 3 Gew.-%, bezogen auf die Masse des Gels, mindestens eines Viskositätsänderungsmittel, das, wenn es mindestens einem Stimulus ausgesetzt wird, der vorzugsweise außerhalb des Gels liegt, eine Veränderung der Viskosität des Gels bewirkt,
wobei der Stimulus, vorzugsweise außerhalb des Gels, aus chemischen Stimuli ausgewählt wird wie eine Änderung des Oxidationsreduktionspotenzials des Gels; thermischen Stimuli wie eine Änderung der Geltemperatur; physikalischen Stimuli wie eine Exposition gegenüber einer elektromagnetischen Welle; und Kombinationen von zwei oder mehr dieser Stimuli,
wobei das Viskositätsänderungsmittel kein superabsorbierendes Polymer ist,
wobei das Viskositätsänderungsmittel aus der Gruppe ausgewählt ist, die aus Polysacchariden, oxidierenden Verbindungen, magnetischen Partikeln und Verbindungen besteht, deren Viskosität variiert, wenn sie elektromagnetischer Strahlung unterschiedlicher Wellenlänge ausgesetzt sind;
- 0,1 bis 10 mol/I Gel, vorzugsweise 0,5 bis 10 mol/l Gel, vorzugsweise noch 1 bis 10 mol/l Gel, von mindestens einem Dekontaminationswirkstoff, der sich von dem Viskositätsänderungsmittel unterscheidet;
- 0,1 Gew.-% bis 2 Gew.-%, bezogen auf die Masse des Gels, mindestens eines oberflächenaktiven Mittels und vorzugsweise mindestens eines nichtionischen oberflächenaktiven Mittels;
- eventuell 0,05 Gew.-% bis 5 Gew.-%, vorzugsweise 0,05 Gew.-% bis 2 Gew.-% in Bezug auf die Masse des Gels, von mindestens einem superabsorbierenden Polymer;
- und das restliche Lösungsmittel.

2. Dekontaminationsgel nach Anspruch 1, wobei das anorganische Viskositätsmittel ausgewählt ist aus Aluminiumoxiden, Kieselsäuren, Aluminosilicaten, Tonen und Mischungen davon; das anorganische Viskositätsmittel ist vorzugsweise ausgewählt aus pyrogenen Kieselsäuren, gefällten Kieselsäuren, hydrophilen Kieselsäuren, hydrophoben Kieselsäuren, sauren Kieselsäuren, basischen Kieselsäuren und Gemischen davon; noch bevorzugter ist das anorganische Viskositätsmittel ein Gemisch aus einer gefällten Kieselsäure und einer pyrogenen Kieselsäure.

3. Dekontaminationsgel nach Anspruch 2, wobei das anorganische Viskositätsmittel aus einem oder mehreren Aluminiumoxid(en) besteht, das/die 5 bis 30 Gew.-%, vorzugsweise 8 bis 17 Gew.-%, bezogen auf die Masse des Gels, ausmacht/ausmachen.

4. Dekontaminationsgel nach Anspruch 1, wobei das Viskositätsänderungsmittel aus Carrageen ausgewählt ist.

5. Dekontaminationsgel nach einem der vorhergehenden Ansprüche, wobei der Dekontaminationswirkstoff aus Basen wie Natriumhydroxid, Kaliumhydroxid und Mischungen davon ausgewählt ist; Säuren wie Salpetersäure, Phosphorsäure, Salzsäure, Schwefelsäure, Wasserstoffoxalate wie Natriumhydrogenoxalat, und deren Gemische; Oxidationsmittel wie Peroxide, Permanganate, Persulfate, Ozon, Hypochlorite, Ceriumsalze IV und deren Mischungen; quaternäre Ammoniumsalze wie Hexacetylpyridiniumsalze wie Hexacetylpyridiniumchlorid; Reduktionsmittel; und deren Mischungen; das superabsorbierende Polymer ist ausgewählt aus Poly(meth)-Natriumacrylaten, Stärken, die mit einem (Meth)-Acrylpolymer transplantiert sind, hydrolysierte Stärken, die mit einem (Meth-)Acrylpolymer transplantiert sind; Polymere auf Stärke-, Gummi- und Zellulosederivatbasis; und deren Mischungen; das nichtionische oberflächenaktive Mittel ist ausgewählt aus Blockcopolymeren, sequenziert wie sequenzierte Copolymere von Ethylenoxid und Propylenoxid, und ethoxylierten Fettsäuren; und deren Mischungen; und das Lösungsmittel ist ausgewählt aus Wasser, organischen Lösungsmitteln und deren Mischungen.

6. Verfahren zur Dekontamination von mindestens einer Oberfläche eines Teils aus einem festen Material, wobei die Oberfläche mit mindestens einer Kontaminationsart kontaminiert ist, die sich auf der Oberfläche und eventuell unter der Oberfläche in der Tiefe des Teils befindet, wobei mindestens ein Zyklus durchgeführt wird, der die folgenden aufeinanderfolgenden Schritte umfasst:
a) das Teil in ein Gelbad nach einem der Ansprüche 1 bis 5 eingetaucht wird, in flüssigem Zustand mit einer Viskosität von weniger als 0,1 Pa.s;
b) das mit einem Gelfilm beschichtete Teil wird aus dem Bad genommen und sofort mindestens einem Stimulus ausgesetzt, der innerhalb von weniger als 5 Sekunden zu einer Erhöhung der Viskosität des Gels und dessen Übergang in einen gelierten Zustand mit einer Viskosität von mehr als 100 Pa.s führt;
c) das Gel mindestens so lange auf der kontaminierten Oberfläche gehalten wird, dass das Gel die Kontaminationsart zerstört und/oder inaktiviert und/oder absorbiert und dass das Gel trocknet und einen trockenen, festen Rückstand bildet, der die Kontaminationsart enthält;
d) der trockene, feste Rückstand, der diese Kontaminationsart enthält, wird entfernt.

7. Verfahren nach Anspruch 6, wobei der Stimulus unter chemischen Stimuli wie einer Änderung des Oxidationsreduktionspotenzials des Gels, thermischen Stimuli wie einer Änderung der Geltemperatur, physikalischen Stimuli wie einer Exposition gegenüber einer elektromagnetischen Welle und Kombinationen von zwei oder mehr dieser Stimuli ausgewählt ist; vorzugsweise ist der besagte Stimulus eine Temperaturabsenkung, die beispielsweise durch den Übergang des Teils von einem beheizten Bad, beispielsweise bei einer Temperatur von 30°C bis 80°C, in die Umgebungsatmosphäre, beispielsweise bei einer Temperatur von 15°C bis 25°C, verursacht wird; oder der besagte Stimulus ist der Beginn der Exposition des Gels gegenüber einem Magnetfeld oder das Stoppen der Exposition des Gels gegenüber einem Magnetfeld.

8. Verfahren nach Anspruch 6 oder 7, wobei der Viskositätsanstieg des Gels in Schritt b) in einer Zeit von weniger als 2 Sekunden, vorzugsweise weniger als oder gleich 1 Sekunde, beispielsweise in einer Zeit zwischen 0,5 Sekunden und 1 Sekunde auftritt.

9. Verfahren nach einem der Ansprüche 6 bis 8, wobei das Teil ein kleines und/oder komplex geformtes Teil ist.

10. Verfahren nach einem der Ansprüche 6 bis 9, wobei das feste Material aus Metallen und Metalllegierungen wie Edelstahl, Aluminium und Blei ausgewählt ist; Polymere wie Kunststoffe oder Gummi wie Poly(vinylchlorid) oder PVC, Polypropylen oder PP, Polyethylen oder PE, insbesondere Polyethylene hoher Dichte oder HDPE, Poly(methylmethacrylat) oder PMMA, Poly(vinylidenfluorid) oder PVDF, Polycarbonate oder PC; Glas; Zement und zementäre Materialien; Mörtel und Beton; Gips; Ziegel; Natur- oder Kunststein; Keramik.

11. Verfahren nach einem der Ansprüche 6 bis 10, wobei die Kontaminationsart unter chemischen, biologischen, nuklearen oder radioaktiven Kontaminationsarten ausgewählt ist, beispielsweise ist die Kontaminationsart eine biologische Art, die unter Bakterien, Pilzen, Hefen, Viren ausgewählt wird, Toxine, Sporen und Protozoen, insbesondere die biologische Art wird unter biotoxischen Arten wie pathogenen Sporen wie beispielsweise Bacillus anthracis-Sporen, Toxinen wie beispielsweise Botulinumtoxin und Viren ausgewählt.

12. Verfahren nach einem der Ansprüche 6 bis 11, wobei das Gel im Verhältnis von 100 g bis 2000 g Gel pro m² Oberfläche, vorzugsweise von 500 g bis 1500 g Gel pro m², vorzugsweise noch von 600 g bis 1000 g Gel pro m² Oberfläche auf die Oberfläche aufgetragen wird.

13. Verfahren nach einem der Ansprüche 6 bis 12, wobei in Schritt c) die Trocknung bei einer Temperatur von 1°C bis 50°C, vorzugsweise von 15°C bis 25°C und bei einer relativen Luftfeuchtigkeit von 20 % bis 80 %, vorzugsweise von 20 % bis 70 % durchgeführt wird.

14. Verfahren nach einem der Ansprüche 6 bis 13, wobei das Gel für eine Dauer von 2 bis 72 Stunden, vorzugsweise 2 bis 48 Stunden, vorzugsweise noch 5 bis 24 Stunden auf der Oberfläche gehalten wird.

15. Verfahren nach einem der Ansprüche 6 bis 14, wobei der trockene und feste Rückstand in Form von Partikeln, beispielsweise Glitter, mit einer Größe von 1 bis 10 mm, vorzugsweise von 2 bis 5 mm, vorliegt.

16. Verfahren nach einem der Ansprüche 6 bis 15, wobei der trockene und feste Rückstand durch Bürsten und/oder Absaugen von der festen Oberfläche entfernt wird.

17. Verfahren nach einem der Ansprüche 6 bis 16, wobei der beschriebene Zyklus 1 bis 10 Mal unter Verwendung des gleichen Gels in allen Zyklen oder unter Verwendung verschiedener Gels in einem oder mehreren Zyklus/Zyklen wiederholt wird.

18. Verfahren nach einem der Ansprüche 6 bis 17, wobei in Schritt c) das Gel vor der vollständigen Trocknung mit einer Lösung eines Dekontaminationsmittels, vorzugsweise mit einer Lösung des aktiven Dekontaminationsmittels des Badgels aus Schritt a) in das Lösungsmittel dieses Gels eingeweicht wird.

## Claims

1. Decontamination gel, consisting of a colloidal solution comprising or consisting of:
- 0.1% to 30% by mass, preferably 0.1% to 25% by mass, preferably again 5% to 25% by mass, better 8% to 20% by mass, relative to the mass of the gel, of at least one inorganic viscosifying agent;
- 0.01% to 5% by mass, preferably 0.05% to 3% by mass, relative to the mass of the gel, of at least one viscosity variation agent which, when subjected to at least one stimulus, preferably external to the gel, causes a variation in the viscosity of the gel,
said stimulus, preferably external to the gel, being selected from chemical stimuli such as a variation in the oxidation-reduction potential of the gel; thermal stimuli such as a variation in the temperature of the gel; physical stimuli such as exposure to an electromagnetic wave; and combinations of two or more of said stimuli,
said viscosity variation agent being not a superabsorbent polymer,
said viscosity variation agent being selected from the group consisting of polysaccharides, oxidizing compounds, magnetic particles, and compounds the viscosity of which varies when exposed to electromagnetic radiation of different wavelengths;
- 0.1 to 10 mol/L of gel, preferably 0.5 to 10 mol/L of gel, preferably again 1 to 10 mol/L of gel, of at least one decontamination active agent different from said viscosity variation agent;
- 0.1% to 2% by mass relative to the mass of the gel, of at least one surfactant and preferably of at least one non-ionic surfactant;
- optionally 0.05% to 5% by mass, preferably 0.05% to 2% by mass relative to the mass of the gel, of at least one super-absorbent polymer;
- and the rest of solvent.

2. Decontamination gel according to claim 1, wherein the inorganic viscosifying agent is selected from aluminas, silicas, aluminosilicates, clays, and mixtures thereof; preferably the inorganic viscosifying agent is selected from pyrogenic silicas, precipitated silicas, hydrophilic silicas, hydrophobic silicas, acidic silicas, basic silicas, and mixtures thereof; preferably again, the inorganic viscosifying agent consists of a mixture of precipitated silica and pyrogenic silica.

3. Decontamination gel according to claim 2, wherein the inorganic viscosifying agent consists of one or more alumina(s) representing from 5% to 30% by mass, preferably from 8% to 17% by mass relative to the mass of the gel.

4. Decontamination gel according to claim 1, wherein the viscosity variation agent is selected from carrageenans.

5. Decontamination gel according to any one of the preceding claims, wherein the decontamination active agent is selected from bases such as sodium hydroxide, potassium hydroxide, and mixtures thereof; acids such as nitric acid, phosphoric acid, hydrochloric acid, sulfuric acid, hydrogen oxalates such as sodium hydrogen oxalate, and mixtures thereof; oxidizing agents such as peroxides, permanganates, persulfates, ozone, hypochlorites, cerium(IV) salts, and mixtures thereof; quaternary ammonium salts such as hexacetylpyridinium salts such as hexacetylpyridinium chloride; reducing agents; and mixtures thereof; the superabsorbent polymer is selected from sodium poly(meth)acrylates, starches grafted with a (meth)acrylic polymer, hydrolyzed starches grafted with a (meth)acrylic polymer; starch, gum, and cellulose derivative based polymers; and mixtures thereof; the nonionic surfactant is selected from block copolymers, sequenced as ethylene oxide and propylene oxide sequenced copolymers, and ethoxylated fatty acids; and mixtures thereof; and the solvent is selected from water, organic solvents, and mixtures thereof.

6. Method for decontaminating at least one surface of a part made of a solid material, said surface being contaminated by at least one contaminant species located on said surface and optionnaly under said surface in the depth of the part, wherein at least one cycle is carried out comprising the following successive steps:
a) the part is immersed in a bath of the according to any one of claims 1 to 5, in the liquid state with a viscosity of less than 0.1 Pa.s;
b) the part, coated with a film of the gel, is removed from the bath and immediately subjected to at least one stimulus that causes an increase in the viscosity of the gel in a time of less than 5 seconds and its transition to a gelled state with a viscosity greater than 100 Pa.s;
c) the gel is maintained on the contaminated surface for at least a sufficient time for the gel to destroy and/or inactivate and/or absorb the contaminant species, and for the gel to dry and form a dry solid residue containing said contaminant species;
d) the dry solid residue containing said contaminant species is removed.

7. Method according to claim 6, wherein said stimulus is selected from chemical stimuli such as a variation in the oxidation-reduction potential of the gel; thermal stimuli such as a variation in the temperature of the gel; physical stimuli such as exposure to an electromagnetic wave; and combinations of two or more of said stimuli; preferably said stimulus is a decrease in temperature, caused for example by the passage of the part from a heated bath, for example at a temperature of 30°C to 80°C, to the ambient atmosphere, for example at a temperature of 15°C to 25°C; or said stimulus is either the beginning of the exposure of the gel to a magnetic field or the stopping of the exposure of the gel to a magnetic field.

8. Method according to claim 6 or 7, wherein the increase in viscosity of the gel during step b) occurs in a time of less than 2 seconds, preferably less than or equal to 1 second, for example in a time of between 0.5 second and 1 second.

9. Method according to any one of claims 6 to 8, wherein the part is a part of small size and/or complex shape.

10. Method according to any one of claims 6 to 9, wherein the solid material is selected from metals and metal alloys such as stainless steel, aluminum and lead; polymers such as plastics or rubbers such as polyvinyl chloride or PVC, polypropylenes or PP, polyethylenes or PE especially high density polyethylenes or HDPE, polymethyl methacrylates or PMMA, poly(vinylidene fluoride) or PVDF, polycarbonates or PC; glasses; cements and cementitious materials; mortars and concretes; plaster; bricks; natural or artificial stone; ceramics.

11. Method according to any one of claims 6 to 10, wherein the contaminant species is selected from chemical, biological, nuclear or radioactive contaminant species, for example the contaminant species is a biological species chosen from bacteria, fungi, yeasts, viruses, toxins, spores and protozoa, in particular the biological species is selected from among biotoxic species such as pathogenic spores such as for example *Bacillus anthracis* spores, toxins such as for example botulinum toxin, and viruses.

12. Method according to any one of claims 6 to 11, wherein the gel is applied to the surface at a rate of 100 g to 2000 g of gel per m² of surface, preferably from 500 g to 1500 g of gel per m², preferably again from 600 g to 1000 g of gel per m² of surface.

13. Method according to any one of claims 6 to 12, wherein, during step c), the drying is performed at a temperature of 1°C to 50°C, preferably from 15°C to 25°C, and at a relative humidity of 20% to 80%, preferably from 20% to 70%.

14. Method according to any one of claims 6 to 13, wherein the gel is held on the surface for a period of 2 to 72 hours, preferably 2 to 48 hours, preferably still 5 to 24 hours.

15. Method according to any one of claims 6 to 14, wherein the dry solid residue is in the form of particles, for example flakes, of a size from 1 to 10 mm, preferably from 2 to 5 mm.

16. Method according to any one of claims 6 to 15, wherein the dry solid residue is removed from the solid surface by brushing and/or vacuuming.

17. Method according to any one of claims 6 to 16, wherein the described cycle is repeated 1 to 10 times using the same gel during all cycles or using different gels during one or more cycle(s).

18. Method according to any one of claims 6 to 17, wherein, during step c), the gel, before complete drying, is soaked with a solution of a decontamination agent, preferably with a solution of the active decontamination agent of the gel of the bath of step a) in the solvent of this gel.
